# EUROPEAN PATENT APPLICATION

(11) **EP 4 533 954 A1**
(43) Date of publication of application: **09.04.2025**
(21) Application number: 23306702.4
(22) Date of filing: 04.10.2023
(51) Int. Cl.: A23C 20/00, A23J 1/00, C07K 14/47, C12N 1/20, C12N 15/00, C12N 15/03

(54) **METHOD FOR PRODUCING CASEIN AND USES THEREOF**

(71) Applicant: Standing Ovation, 75014 Paris (FR)
(72) Inventor: CHAYOT, Romain, 75014 Paris (FR); DIALLO, Mamou, 91210 Draveil (FR)
(74) Representative: Flesselles, Bruno F.G.

(57) **Abstract**

The invention pertains to the food industry and relates to new methods for producing casein compositions by culturing transgenic microorganisms in presence of lactoserum, and limiting the environmental footprint.

## Description

The invention pertains to the food industry and relates to new methods for producing casein compositions and uses thereof, in particular for producing dairy substitute (notably vegan cheese), while limiting the environmental footprint.

### Background

The use of milk as a protein, sugar and lipid-rich nutrient, has become almost universal in traditional societies. In addition, milk transformation into various derivatives to obtain dairy products, is one of the most ancient examples of human agro-industries. Today, the cheese market represents 20 million tons of product per year, for a value of about $140bn.

However, dairy products, are associated with several issues or concerns, in terms of health, as well as environmental and ethical considerations. These concerns have highlighted the need for dairy product substitutes alleviating these various issues. Health issues include lactose intolerances, allergies, and others, such as a high content in saturated fatty acids, known to have a potential negative impact on health.

In addition, environmental and ethical concerns related to food products of animal origin have raised over the last decades. The burden of animal breeding for a 7 billion population (11 billion in 2050) has become increasingly high. The environmental consequences are severe. They are today mostly considered in terms of anthropogenic greenhouse gas (GHG) emissions, water consumption, pollution by effluents, and land occupation.

Cattle breeding is today considered as one of the first sources GHG emissions, with an estimated 7.1 Gigatonnes of CO2-equivalent per year, representing 14,5% of all anthropogenic greenhouse gas (GHG) emissions.

A very strong water consumption is also associated with animal farming. In addition, farming effluents also have a strong environmental impact. Although the impact of crop fertilizers on groundwater should not be neglected, the impact of animal farming effluents is often massive and has proven to be disastrous in many parts of the world. Furthermore, livestock takes up to nearly 80% of global agricultural land, yet produces less than 20% of the world's supply of calories, showing the very high level of stress brought by animal farming on land resources.

Finally, animal welfare has become an increasingly important concern. The scale-up of meat and dairy production and processing has turned it into an intensive industrial process, which is increasingly perceived as ethically not acceptable.

Therefore, there is a strong need for dairy products substitutes alleviating the issues listed above.

Plant-based substitutes are a potential alternative to conventional dairy products. However, these products, which are often derived from starch, soy, almond, or coconut milk, can be far from mimicking the taste of dairy products. In addition, they are anyway fundamentally different in terms of composition.

Therefore, there is today a need for dairy substitutes, and in particular, cheese substitutes that are
(i) free of undesired compounds
(ii) similar to the original product in aspect, texture and taste
(iii) equivalent or superior to the original products in terms of nutrition

Cows' milk typical composition is described in Table 1 and more detailed compositions of cow's and other animals milk, including the listing of the different lipids, proteins, salts, vitamins and other nutrients can be found from a large number of sources (https://en.wikipedia.org/wiki/Milk#Cow's_milk; Haug et al (2007) Bovine milk in human nutrition - a review, Lipids Health Dis.; Volume 6, pages 25; Dominguez-Salasa et al (2019) Contributions of Milk Production to Food and Nutrition Security; Encyclopedia of Food Security and Sustainability, Volume 3, pages 278-291).

**Table 1: Cow's milk composition**

| Type of molecule | mass in 1 liter in grams |
|---|---|
| Water | 902 |
| Lactose | 49 |
| Fat | 39 |
| Caseins | 28 |
| Other proteins | 5 |
| Salts and vitamins | 9 |
| Total | 1032 |

Lipids and carbohydrates (but lactose) can be recovered from plant, calcium from inorganic sources, from animal sources or from plants (such as sea weeds). In the case of proteins, other sources need be considered as animal proteins differ in amino-acid content from plant proteins. Milk's proteins can also be produced by fermentation, another source of ingredients of a non-animal origin.

Furthermore, proteins isolated form milk are also individually used as nutritional supplements and other applications. Therefore, milk proteins made by fermentation, independently of other milk components can also be used for other applications than the making of dairy substitutes.

Production by fermentation is based on the growth of microorganisms (bacteria or fungi) producing a compound of interest in a fermenter, usually followed by the recovery and purification of the compound of interest. Such microorganisms are generally transgenic, *i.e.* transformed with a genetic construct for expression of the desired protein(s) or compound obtained by metabolism of the microorganisms. The making of proteins by fermentation is a process that has widely been used in the food industry. Several studies have described the production of milk's constitutive proteins or homologues by fermentation in various microorganisms (see below) and the assembly of ingredients including fermentation proteins to make dairy substitutes has been described several times (US6270827; US5,942,274; WO2018039632; WO2020223700; WO2020081789, WO2020219596, WO2022098835).

Production by fermentation us usually conducted with a sugar feedstock, to support the growth of the microorganism and the production of the desired compound. Depending on the capacity of the microbe to metabolize the different sugars, one can use glucose or sucrose or other sugars such as lactose.

For example *Escherichia coli* is known to consume glucose very efficiently, but can also consume a series of other sugars, including glucose, maltose, lactose, trehalose, fructose, xylose and arabinose with a hierarchy of preference.

Microorganism growth and metabolism requires not only sugar feedstock, nut also nitrogen input. For this, various compounds can be added, including liquid or gaseous ammonia, urea, amino acids, peptones, and yeast extract.

Alternative feedstocks derived from dairy, such as lactoserum (whey)have also been used in fermentation with various microorganisms comprising *Escherichia coli* (González-Siso (1996) The biotechnological utilization of cheese whey: A review. Bioresource technology vol. 57, no 1, p. 1-11; Viitanen et al. (2003), Cheese whey-induced high-cell-density production of recombinant proteins in Escherichia coli. Microbial Cell Factories volume 2:2; Guimaraes et al. (2010) Fermentation of lactose to bio-ethanol by yeasts as part of integrated solutions for the valorisation of cheese whey. Biotechnology Advances 28: 375-384; Christensen et al. (2011) Production of bioethanol from organic whey using Kluyveromyces marxianus. J Ind Microbiol Biotechnol volume 38, pages 283-289; Pasotti et al. (2017) Cheese whey-induced high-cell-density production of recombinant proteins in Escherichia coli BMC Biotechnol. 17:48; Amaro et al. (2019) Prospects for the Use of Whey for Polyhydroxyalkanoate (PHA) Production. Front. Microbiol. Volume 10: 992-; Hausjell et al. (2019) Valorisation of cheese whey as substrate and inducer for recombinant protein production in E. coli HMS174(DE3). Bioresource Technology Reports volume 8:100340; Louasté and Elourtassi (2020) Succinic acid production from whey and lactose by Actinobacillus succinogenes 130Z in batch fermentation. Biotechnology Reports volume 27:e00481; Carranza-Saavedra et al. (2021), Kinetic analysis and modeling of L-valine production in fermentation batch from E. coli using glucose, lactose and whey as carbon sources. Biotechnology Reports volume 31, e00642; Chaparro et al. (2021), Whey as an Alternative Nutrient Medium for Growth of Sporosarcina pasteurii and Its Effect on CaCO3 Polymorphismand Fly Ash Bioconsolidation. Materials volume 14: 2470; Mobayed et al. (2021) Effect of by-products from the dairy industry as alternative inducers of recombinant β-galactosidase expression. Biotechnol. Letter 43(3):589-599; Zou and Chang (2022) Past, Present, and Future Perspectives on Whey as a Promising Feedstock for Bioethanol Production by Yeast. Journal of Fungi, volume 8, pages 395-; de Diviitis et al. (2023) Cheese-whey permeate improves the fitness. Biotechnology for Biofuels and Bioproducts, volume 16:30).

Lactoserum is a byproduct or waste of the cheese-making process, but also any other process, such as Greek yoghourt production, wherein some whey is drained out of the product after curdling. Whereas cheese production can result in 10L of lactoserum for 1 kg of cheese (Mollea et al. (2013) Chapter 24: Valorisation of cheese whey, a by-product from the dairy industry. Pages 549-588 in Food Industry. I. Mazzalupo, ed. InTech Open, London, UK), production of 1 kg of Greek yoghourt can result in about 2 to 3 kg of acid whey (Erickson, B. E. (2017) Chem. Eng. News, 95(6), 26-30).

The valorization of lactoserum, and notably acid lactoserum is an important issue in the dairy industry (Gonzales-Siso (1996) The biotechnological utilization of cheese whey: a review. Bioresour Technol. 57:1-11; Marwaha and Kennedy (2007) Whey-pollution problem and potential utilization. Int J Food Sci Technol.;23:323-36; Rocha-Mendoza et al (2021) Acid whey trends and health benefits. J. Dairy Sci. volume 104, pages 1262-1275).

Lactoserum contains proteins (the whey proteins, about 20% of total milk's proteins), and has a complex composition including minerals and lactose. Whey is characterized by its green-yellowish color, resulting from the presence of riboflavin (vitamin B12; González-Siso (1996) The biotechnological utilization of cheese whey: A review. Bioresource technology vol. 57, no 1, p. 1-11;). There are two main types of lactoserum: sweet lactoserum and acid lactoserum.

Sweet lactoserum is a byproduct associated with most cheese production, after enzymatic curdling using natural or synthetic rennet. It has a pH around 6 to 7 (González-Siso (1996) The biotechnological utilization of cheese whey: A review. Bioresource technology vol. 57, no 1, p. 1-11), and has a higher protein content and lower salts than acidwhey

Acid lactoserum is associated with acid curdling of milk. Acidic curdling can result from the activity of lactic bacteria, lowering the by the pH by the production of organic acids, or from the addition of organic (lactic, citric, acetic) or mineral (sulfuric, chlorhydric) acids. Acid whey is a natural by product of cottage cheese and Greek yoghourt two products production has increased significantly. Its valorization is difficult for several inherent reasons (Rocha-Mendoza et al (2021) Acid whey trends and health benefits. J. Dairy Sci. volume 104, pages 1262-1275), and its use in fermentation represents a solution to address this issue.

Lactoserum can vary in composition, depending on the production process. For example, in acid lactoserum, lactose concentration can vary from about 2 to about 5% (weight/volume) (Rocha-Mendoza et al (2021) Acid whey trends and health benefits J. Dairy Sci. volume 104, pages 1262-1275; O'Donoghue and Murphy (2023) Comprehensive Reviews in Food Science and Food Safety, 22:2652-2677).

Various whey derivatives may be obtained, in order to valorize whey proteins and lactose.

Lactoserum permeate is obtained after lactoserum proteins are removed using ultrafiltration (O'Donoghue and Murphy (2023) *op.cit.*)*.* Lactoserum permeate can be used as a source of dairy solids in various food, beverage, or feed applications.

In delactosed lactoserum permeate, a large part of lactose is removed, although lactose can still represent 60% of dry weight, and thus remain a significant part of the remaining composition.

Lactoserum can also be concentrated, in order to facilitate transportation.

We will refer indifferently to such products as lactoserum or whey, whether they are raw (or whole) lactoserum, permeates, or delactosed permeates, as long as they still contain at least about 2% of lactose (w/w, wet weight), preferably at least 3%, preferably at least 4%, preferably at least 5%, preferably at least 8%. In some embodiments, and when concentrated lactoserum is used, it may contain at least 30% lactose (w/w wet weight), or at least 32% lactose or 35% lactose or more.

Using this type of feedstock does not qualify for the making of animal-free products, given the origin of whey. Nevertheless, the use of a waste as feedstock can be very helpful to reach good economics and advantageous life cycle analysis.

During fed-batch fermentation, pH can be adapted to keep within a certain range, and for this addition of acidic or basic compounds can be used, including hydrochloric acid, sulfuric acid, lactic acid, and phosphoric acid (acidic compounds) or liquid or gaseous ammonia, sodium hydroxyde, or potassium acid (basic compounds).

All these solutions are not exclusive and can be combined.

The inventors have proposed to use lactoserum as a source of carbon for the culture of microorganisms expressing caseins, in particular during fed-batch culture. When such product is used, the inventors have shown that the environmental footprint is decreased as compared to the footprint observed when using glucose, sucrose or sucrose molasses as the feedstock for microorganisms growth (with Isopropyl β-d-1-thiogalactopyranoside (IPTG) used to induce casein production if appropriate).

It is reminded that a fed-batch process is a method for the cultivation of microorganisms, such as bacteria, yeast, or cells, in a bioreactor to produce substances of interests. This process offers greater control over the culture environment and can lead to higher yields and product concentrations compared to batch cultures.

The process begins with the inoculation of a small quantity of microorganisms (the inoculum) into the bioreactor containing a growth medium. This medium contains essential nutrients (such as sugars, nitrogen sources, minerals, and vitamins) for the microorganisms to grow and produce the desired product.

The bioreactor initially operates in a batch mode, with all the necessary nutrients are added at the beginning of the cultivation. During this phase, the microorganisms grow, consume nutrients, and may also produce the target product. The culture medium doesn't contain an excess of nutrients, to avoid overgrowth, production of deleterious by-products by the microorganisms, and to ensure better control.

As the culture progresses, the concentration of nutrients in the bioreactor decreases, upon consumption by the microorganisms. Additional nutrients are gradually introduced into the bioreactor during the fed-batch phase. The rate and amount of nutrient addition as well as various parameters such as temperature, pH, dissolved oxygen, agitation speed, and nutrient feed rates are closely monitored, controlled, and adjusted as needed to optimize cell growth and product formation based on factors like cell density, nutrient levels, and product formation, to maintain optimal growth conditions and extend the production phase. In continuous fed-batch processes, nutrients are continuously provided. In other fed-batch processes, nutrients are provided from time to time.

Once the desired product concentration is achieved or after a given period of time, the culture is harvested. Harvesting is performed before decrease of the amount of microorganisms, as death of such may lead to formation of undesired products. The product can be purified and further processed.

The advantages of a fed-batch process include: controlled growth of the microorganisms and product formation by adjusting nutrient feed rates, higher yields by providing nutrients in a controlled manner, extended production phase, reduced byproduct formation.

In one embodiment, lactoserum is present in the culture medium at the start of the culture (inoculation phase), alone or preferably with another sugar, notably glucose, saccharose, fructose, in particular in the form of molasse.

In another embodiment, lactoserum is not present in the culture medium at the start of the culture (inoculation phase). In this embodiment, another sugar is present in the culture medium, notably glucose, saccharose, fructose, in particular in the form of molasse.

In one embodiment, lactoserum is introduced as the carbon source during at least part of the fed-batch period. In one embodiment, no other sugar is provided with lactoserum. In another embodiment, lactoserum is introduced with another sugar, as indicated above.

In one embodiment, lactoserum is present in the starter culture medium together with a sugar other than lactose (notably glucose), and the fed-batch process comprises provision of additional nutrients, wherein no sugar is provided with the additional nutrients for a least a period of time (ranging from 15 minutes to 1 hour, preferably from 20 minutes to 45 minutes, preferably around 30 minutes), and a sugar another than lactose (notably glucose) is further provided again with the additional nutriments, after this period of time. In one embodiment, lactoserum is not further provided with the sugar another than lactose (notably glucose). In another embodiment, lactoserum is further provided with the sugar another than lactose (notably glucose).

In one embodiment, lactoserum is not present in the starter culture medium, which contains only a sugar another than lactose (notably glucose). In this embodiment, the fed-batch process comprises provision of additional nutrients, wherein lactoserum is the only sugar is provided with the additional nutrients for a least a period of time (ranging from 15 minutes to 1 hour, preferably from 20 minutes to 45 minutes, preferably around 30 minutes). In this embodiment, a sugar another than lactose (notably glucose) may be further provided again with the additional nutriments, after this period of time. In one embodiment, lactoserum is not further provided with the sugar another than lactose (notably glucose). In another embodiment, lactoserum is further provided with the sugar another than lactose (notably glucose).

One goal is to use the lactose present in the lactoserum as the sole sugar to be used by the microorganisms for some time during cell culture. When expression of the casein is induced by lactose, this also ensures the switch to casein production by the microorganisms. When the production of the caseins has started, it is possible to reintroduce glucose or another sugar (even lactose as a sugar).

In view of the concentration of lactose in the lactoserum that is not concentrated, one favorable embodiment is to use a medium containing lactoserum (up to 60%, preferably up to 55%, in particular around 45% to 55%, notably around 50% v/v) as the starter medium, and also containing a sugar other than lactose, that induces CCR (see below), such as glucose. The microorganisms shall then use and consume the other sugar. At a time specified by one of skill in the art (generally when the microorganisms are in the second half of the exponential growth phase), no further sugar other than lactose is provided, so that the microorganisms will consume the lactose present in the medium from the start of the culture (which was not consumed as the other sugar was used by the microorganisms as the source of carbon). Lactose catabolism thus starts and CCR is lifted (thus allowing consumption of the lactose present in the medium and induction of casein production when it is lactore inducible). Lactose is then consumed, and further carbon provision can be obtained by feeding with any sugar (glucose, lactose, fructose, saccharose), without further provision of lactoserum.

In some embodiments, lactoserum is used as the only source of sugar (lactose present in the lactoserum).

In other embodiments, lactoserum is used with other feedstocks, such as glucose (which can be obtained from corn or wheat), saccharose (available from sugar beets or sugar cane). Such other feedstocks may be in the form of pure sugar, molasses or others forms.

It is preferred to use lactoserum together with other feed stocks. Indeed, due to the fact that concentration of lactose in lactoserum is generally low, using such product as the sole feedstock would require addition of a large volume of lactoserum to obtain the adequate lactose amount.

In addition, since lactoserum contains lactose, it can trigger expression of the transgenes expressing the caseins, when a lactose-inducible promoter is used (Viitanen et al. (2003), Cheese whey-induced high-cell-density production of recombinant proteins in Escherichia coli. Microbial Cell Factories volume 2:2; Hausjell et al. (2019) Valorisation of cheese whey as substrate and inducer for recombinant protein production in E. coli HMS174(DE3). Bioresource Technology Reports volume 8:100340; Mobayed et al. (2021) Effect of by-products from the dairy industry as alternative inducers of recombinant β-galactosidase expression. Biotechnol. Letter 43(3):589-599; de Diviitis et al. (2023) Cheese-whey permeate improves the fitness. Biotechnology for Biofuels and Bioproducts, volume 16:30;
In one embodiment, it is proposed to use have both glucose and lactoserum in the culture medium early in the growth of the microorganisms (up to the end of the exponential growth or the beginning of the stationary growth), whereas glucose will be preferably used, due to the carbon catabolic repression (CCR), and to switch to lactose catabolism, once the appropriate growth of the microorganisms have been achieved and when expression of the recombinant casein is desired (Görke and Stülke Nat Rev Microbiol 6, 613-624 (2008); Postma et al Microbiol Rev. 1993 Sep;57(3):543-94). It may be possible to feed the culture with a low level of glucose, which will maintain appropriate carbon supply to maintain vigor of the microorganism growth without any switch back to only glucose use, and hence continuing protein expression. Although other sugars could be used (such as sucrose or galactose), use of glucose is preferred, at least at the start of the culture (exponential growth), to induce CCR, in particular when lactose inducible promoters are used to trigger expression of the casein(s).

Lactose is a disaccharide composed of glucose and galactose. Its metabolization generally depends on its hydrolysis in glucose and galactose. Some *Escherichia coli* strains such as BL231(DE3) cannot consume the galactose moiety, because of a mutation that results in the lack of enzymes involved in galactose metabolization by the Leloir pathway. Consequently, in order to fully use lactose as a provider of carbon for the bacteria, with maximal yield, it is possible and preferred to use strains with a proficient Leloir pathway (Hausjell et al. (2019) Valorisation of cheese whey as substrate and inducer for recombinant protein production in E. coli HMS174(DE3). Bioresource Technology Reports volume 8:100340), or to restore this pathway in mutated strains by genetic engineering. Such strains able to metabolize D-galactose and form glucose are generally designed as *gal*+ strains.

Furthermore, the inventors used adapted purification procedures, to produce the caseins at a grade compatible with use in the food industry, with a high level of purity, but are also easily scalable at industrial level, and at costs compatible with exploitation, in particular energy costs.

In particular, recombinant caseins can be isolated from the biomass and from the culture broth, by heating the composition, as disclosed in WO2022253816.

Using the methods herein disclosed herein makes it possible to obtain casein compositions with an environmental footprint that is reduced when compared to the footprint observed when glucose, sucrose or sucrose molasses is used as the carbon source for culturing the microorganisms (i.e. when lactoserum, and especially acid lactoserum or acid lactoserum permeate is not used in the process). In addition, it makes it possible to provide to the dairy industry a way to recycle wastes, which currently must be treated and disposed of. When microorganisms are used with lactose-induced expression of caseins, IPTG is used for inducing expression of the proteins, at the time casein expression would be induced by the lactose of the lactoserum.

Environmental impact is multifactorial and must be considered in terms of climate change, pollution of freshwater, marine water, soils and air, impact on ecosystems, use of fossil resources, depletion of material resources, water use and others. Criteria can include
(i) Climate change, measured in kgCO₂eq (eq for equivalent)
(ii) Acidification, in mol H⁺-eq
(iii) Ecotoxicity, in comparative toxic unit for ecosystems (CTUe)
(iv) Use of non-renewable energy resources (in MJ or KWh)
(v) Eutrophisation of freshwater (in kg P-eq) (kg phosphorus equivalent)
(vi) Eutrophisation of marine water (in kg N-eq) (kg of Nitrogen equivalent)
(vii) Eutrophisation of terrestrial land (accumulated excedence in mol N-eq)
(viii) Human toxicity, carcinogenic (in comparative toxic unit for human, CTUh)
(ix) Human toxicity, non-carcinogenic (in comparative toxic unit for human, CTUh)
(x) Ionizing radiation, impact on human health (human exposure efficiency relative to U₂₃₅ kNq U₂₃₅-eq)
(xi) Land use soil quality index (ss)
(xii) Use of material resources (kg Sb-Eq)
(xiii) Ozone depletion potential (in kg CFC11-eq)
(xiv) Particulate matter formation (disease incidence)
(xv) Photochemical oxidant formation (kg NMVOC-eq)
(xvi) Water use (m³ world eq. deprived)

The method is of interest to provide improvements for at least one of these criteria, in particular climate change, acidification, eutrophisation (v, vi and/or vii) and/or water use.

The casein composition can also be processed to obtain dairy substitutes, such as cheese, yoghurt, that result from curdling, but also ice cream, reconstituted milk and the like, the environmental footprint of such dairy substitutes being also reduced as compared to the environmental footprint observed when using recombinant caseins from microorganisms grown in presence of glucose, sucrose or sucrose molasses as the carbon source.

In the context of the present invention, a "casein" is any casein protein or mixture of casein protein. Thus, a "casein" is an alpha-S1 casein, an alpha-S2 casein, a beta casein, or a kappa casein. In some extend, it can also designate any mixture of such proteins. One can use the term "casein" or "caseins" to discuss casein proteins in general.

In the context of the invention, the term "between" includes the limits.

In the context of the present invention, the term "dairy substitute" means a food product having the essential features of a dairy product, obtained using cow milk, (such a cheese, yogurt, ice cream...), in terms of nutritional value, aspect, texture and taste.

In the context of the present invention, the term "cheese substitute" means a food product having the essential features of cheese in terms of nutritional value, aspect, texture and taste.

The term "fresh cheese" designates a cheese with a moisture higher than 80% on a fat-free basis (ratio of water vs. total mass of product without fat), and a protein content comprised between 2% and 15% of total weight (ratio of protein mass vs. total product mass).

The term "soft cheese" or "semi-soft cheese" designates a cheese with a moisture comprised between 62% and 80% on a fat-free basis (ratio of water vs. total mass of product without fat), and a protein content comprised between 15% and 30% of total weight (ratio of protein mass vs. total product mass). A soft cheese has a moisture comprised between 67% and 80%, on a fat-free basis, and a semi-soft cheese has a moisture comprised between 62% and 67%, on a fat-free basis.

In the context of the present invention, the term "liquid pre-curd composition" or "LpCC" designates the composition containing at least a casein and at least one other ingredient comprising at least a component among water, calcium, lipids, an carbohydrate, before the addition of rennet and ferments and curdling (in particular coagulation of casein). The casein concentration in the LpCC is higher than that in milk. The concentration of the other ingredient in the LpCC is also higher than that in milk. This makes it possible to save some water when using a LpCC with recombinant caseins as disclosed in the present application, as the LpCC doesn't represent an artificial (cow's) milk. The respective concentrations of proteins, lipids, calcium salts and/or carbohydrates are adjusted to fit the final desired compositions of cheese substitutes

In the context of the present invention, the term "curdling agent" designates a chemical or biochemical composition capable of triggering curdling. Curdling can be achieved by the addition of acid solutions, by the addition of starter cultures (ferments which growth in the presence of carbohydrates triggers a decrease of pH), by heat treatment, by addition of calcium chelating agent, by the addition of natural or recombinant rennet, by the addition of rennet substitutes, such as animal proteases, or vegetal curdling enzymes), or by a combination of these processes. A curdling agent may be any additive, compound, composition, or treatment which addition results in curdling, alone or in combination with another or others additives, compounds, compositions, or treatments. When preparing animal-free edible compositions, it is preferred to use an acidifying agent (such as an acid or starter cultures), in order to avoid using rennet, so that the curdling agent does not comprise any element of animal origin. It is advantageous when the acidifying agent comprises a lactic bacterium or lactic bacteria, but acidic chemicals can also be used.

In the context of the present invention, the term "curd" means a composition wherein casein coagulates or precipitate, under the action of the curdling agent, and which can be separated from a liquid phase, if any, by draining, for example on a cheese cloth. The curd also comprises other ingredients, including water and lipids (when present),

In the context of the present invention, the term "ferment" designates a composition containing at least one microbial strain, added during the process of production of the cheese substitute. Lactic ferments are responsible for lactic fermentation, resulting notably in an acidification of the medium. As a consequence, they can be used as a curdling agent.

Other ferments are used in cheese production for the processing of the curd, resulting in modifications of texture, taste, smell, and chemical composition (with notably the cleavage of proteins into smaller peptides). These ferments can be called "ferments for maturation", "maturation ferments" or "ripening ferments" and are generally not used for production of fresh cheeses. Such ferments for maturation can be added together with the curdling agent.

In the context of the present invention, the term "of non-animal origin" means a compound or composition which has not been directly derived from an animal, produced from animal cells in culture, or isolated from animal products such as milk. Compounds or compositions produced by fermentation of microbes are thus "of non-animal origin" even though some products of animal origin, bacto peptone for example, can be involved during fermentation. Therefore, in the context of the invention, a protein that is naturally produced in animals will be called of non-animal origin when it is produced in microbial (such as bacterial or yeast) cells or in plant cells, even though its sequence or structure may be identical to the sequence or structure of the protein that would be isolated from animal.

In the context of the present invention, the term "animal-free" means a compound or composition which has not been derived from an animal, from animal cells in culture, or from animal products such as milk, and whose production process does not involve any feedstock or additive of animal origin.

In the context of the invention, the term "texturing agent" means any gelling agent, including emulsifier such as lecithin, and hydrocolloids such as cassia gum, sesbania gum, tamarind gum, guar gum, fenugreek gum, Arabic gum, agar agar (or agar-agar), carrageenans, tragacanth gum, xanthan gum, carob (locust bean) gum, cellulose gum.

In a first aspect, is herein disclosed a method for obtaining a casein composition, comprising
i. providing microorganisms that have been transformed with at least one nucleic acid coding for a casein,
ii. culturing said microorganisms, using a medium comprising lactoserum so as to express and produce casein,
iii. thereby providing a microorganism composition wherein the pH of the composition is equal or above 6.5 and preferably below 9
iv. heating the microorganism composition so as to reduce the amount of the other proteins in soluble fraction of the composition, wherein heating is performed at a temperature at a temperature equal or above 75°C,
v. recovering the soluble fraction from the heated cell composition of iv), thereby obtaining a casein composition in the soluble fraction.

During step (ii), lactoserum can be used in combination with other sugars, such as glucose, saccharose, or even lactose.

Said method makes it possible to obtain a reduced environmental footprint for at least one of one of the criteria mentioned above (in particular the Green House Gas (GHG) emissions, in mass of CO₂eq), as compared to performing the process using glucose, sucrose or sucrose molasses in place of lactoserum in (ii). The footprint can be particularly reduced when acid lactoserum (or derivatives, such as permeate) is used as the lactoserum in step (ii).

In particular, it is possible to obtain a reduction of at least 5%, at least 10%, at least 15%, at least 20%, at least 30%, at least 40%, or even at least 50%, depending on the criteria that is looked at.

Reduced environmental footprint can also be observed for dairy substitutes made with caseins obtained by the methods herein disclosed.

It is reminded that microorganisms, in particular bacterial cells present an exponential (or log) phase, in which cells are doubling after each generation time. Metabolic activity is high in this phase, and energy (nutrients) in the culture medium is directed to such doubling activity. After the exponential time, the population growth experienced in the log phase begins to decline as the cell growth reaches a plateau, or stationary phase, where the number of dividing cells equal the number of dying cells.

In the context of the process herein disclosed, it is preferred to have lactoserum, as a feedstock to be used by the microorganisms, preferably in the middle or late exponential phase or at the beginning of the stationary phase (this is when feed supply of a sugar other than lactose is avoided, and feed supply is made with lactoserum or lactoserum is present within the medium from the inoculum phase), and sometimes to maintain this provision during the production of the caseins. However, early induction streps can also be envisioned. One of skill in the art can determine the end of the exponential phase or the start of the stationary phase, for a culture of microorganism, which depends on the doubling time of the microorganisms, the concentration of the starter culture, and the duration of growth. Measuring the Optical Density (OD) of the culture may also provide information as to the growth phase of the microorganism. When *Escherichia coli* is used, as a microorganism, one can envisage a duration of exponential phase of between 16-20 hours, followed by a stationary phase during which expression of the protein is performed of about 7-10 hours.

The microorganism composition is a liquid solution that contains casein and other proteins. It also presents a soluble fraction and a non-soluble fraction. This composition also contains transgenic microorganisms such as bacteria that produce caseins as a result of the presence, in their genome of transgene(s) coding for casein. Such bacteria (whether lyzed or not) are present in the non-soluble fraction.

The composition containing the transgenic microorganism is obtained by fermentation, using various sugars and nitrogen sources as feedstocks. In one embodiment, fermentation is conducted in a medium containing glucose, sucrose or lactose in addition to lactoserum. In a preferred embodiment, glucose, sucrose or lactose are used as a carbon source. In a some embodiments, fermentation is conducted in a medium using molasses or vinasses as a carbon source. In one embodiment, fermentation is conducted in a medium containing molasses as a carbon source. In another embodiment, liquid or gaseous ammonia, urea, amino acids, peptones, or yeast extract are present or added in the medium as a nitrogen source. In one embodiment, fermentation is conducted in a medium containing ammonia, urea, amino acids, peptones, or yeast extract as a nitrogen source. In one embodiment, fermentation is conducted on a medium containing lactoserum as the main feedstock (main source of carbon). In one embodiment, fermentation is conducted in a medium containing acid lactoserum or acid lactoserum permeate. In one embodiment, fermentation is conducted in a medium containing lactoserum and another sugar as additional sugar source. In a preferred embodiment, fermentation is conducted on a medium containing acid lactoserum (or acid lactoserum permeate) and glucose, sucrose or lactose as additional sugar source. Glucose can be used as the other carbon source.

Before heating, or during growth of the microorganisms, pH can be adjusted by the addition of as liquid or gaseous ammonia, sodium hydroxide, potassium hydroxide or calcium hydroxide or other basic compounds. pH is superior to 6,5, and preferably inferior to 9.

The microorganisms are transgenic and contain at least one transgene (a nucleic acid) coding for a casein that is introduced present in the microorganisms. This transgene can be present within the microorganisms' genome, or be present outside of the genome (on a plasmid, a cosmid, an artificial chromosome such as a bacterial artificial chromosome or a yeast artificial chromosome).

In a specific embodiment, the microorganisms are bacterial cells, in particular *Escherichia coli.* In another embodiment, the microorganisms are fungi cells (including yeast cells). In another embodiment, the microorganisms are eukaryotic cells, in particular plant cells. In particular, prokaryotic hosts suitable for expressing caseins include *E. coli, Bacillus subtilis, Salmonella typhimurium, Lactococcus lactis* and various species within the genera *Lactococcus, Pseudomonas, Streptomyces,* and *Staphylococcus.* Eukaryotic host suitable for expressing caseins include fungi, such as *Saccharomyces cerevisae, Kluyveromyces lactis, Pichia pastoris, or Trichoderma reesei*)*.* Plant cells may also be used for producing protein.

It is preferred that the casein is produced in non-animal cells, preferably in bacterial cells.

The microorganisms are transformed with at least one nucleic acid for expression of a casein. Such nucleic acid contains the elements necessary for the transcription and translation of the transgene coding for casein. In particular, the n nucleic acid shall contain a promoter sequence, a sequence coding for casein and a terminator sequence.

In some embodiments, the microorganisms are transformed with one nucleic acid coding for one casein (and produce such casein). It is preferred when expression of the nucleic acid (production of the protein) is induced by lactose, i.e. the nucleic acid coding for the casein is expressed in the presence of lactose metabolized by the microorganism (lactose-induced expression).

In other embodiments, the microorganisms express two caseins. In such embodiments, the microorganisms may be transformed with two transgenes, each for the expression of a given casein. It is preferred when expression of at least one transgene is a lactose-induced expression. Preferably expression of the two transgenes are lactose-induced expression (using identical or different systems). In another embodiments, the two transgenes are linked together in an operon (the caseins are thus simultaneously produced). It is preferred when expression of the operon is a lactose-induced expression.

One also envisages embodiments, where the microorganisms express more than two caseins. In such embodiments, each transgene coding for a given casein may be under the control of its own promoter (preferably for a lactose-induced expression), or part or all of the transgenes may be co-expressed in an operon (preferably for a lactose-induced expression).

In some embodiments, the microorganism culture contains a mixture of some microorganisms transformed with one nucleic acid coding for one casein and of other microorganisms transformed with one nucleic acid coding for another casein.

Lactose induced expression of a gene is known in the art. It is often based on the lactose operon (lac operon), which contains an operator region (lac o), to which binds the lac repressor, lacl,in the absence of lactose, thereby preventing transcription of the genes downstream the *lac* o region. In the presence of lactose (allolactose, isomer of lactose), the conformation of the lacl protein changes and it doesn't bind to *lac* o anymore, thereby allowing transcription of the sequences downstream the operator. The sequence of the lac operator TTGTGAGCGGATAACAA (SEQ ID NO: 5). The system (that comprises the *lac* o operator and the *lac*I repressor) can be used for expression of genes downstream the *lac*o operator, with a promoter upstream the *lac*o operator being either the native promoter of the lac operon, or another promoter (such as the T7 promoter).

One can note that lactose inducible promoters and systems have been described in the art. One can cite the lac promoter that regulate expression of the lactose operon (lac operon), an operon required for the transport and metabolism of lactose, in *E. coli* and many other enteric bacteria. One can also cite the P(bgaL) promoter (Hartman et al, Appl Environ Microbiol. 2011 Jan;77(2):471-8), the P_{lacA} and P_{lacLM} promoters disclosed in Zhang et al (Biochemical Engineering Journal Volume 151, 15 November 2019, 107316), or the P_{lacA} promoter disclosed in Heiis et al (Microb Cell Fact 15, 50 (2016)).

As indicated above, a specific embodiment encompasses the situation when the starting composition (composition containing the casein and other proteins) has been obtained from a bacterial culture. In such embodiment, and as indicated above, the bacteria have been transformed with one or more nucleic acid coding for one or more casein.

In particular, in one embodiment, the bacteria have been transformed with one or more nucleic acid coding for a beta casein.

In particular, in one embodiment, the bacteria have been transformed with one or more nucleic acid coding for an alpha-S1 casein.

In particular, in one embodiment, the bacteria have been transformed with one or more nucleic acid coding for an alpha-S2 casein.

In particular, in one embodiment, the bacteria have been transformed with one or more nucleic acid coding for a beta casein and an alpha-S1 casein.

In particular, in one embodiment, the bacteria have been transformed with one or more nucleic acid coding for a beta casein, an alpha-S1 casein and an alpha-S2 casein, or a combination of two out of them.

It is to be noted that, when multiple proteins are produced, the bacteria can be transformed with different nucleic acids (each one coding for a different protein) or with a unique nucleic acid (which contains the elements allowing production of the various proteins, such as operon). Such methods of transforming microorganisms and bacteria in particular, for production of one or of multiple proteins are known in the art.

Consequently, the recovered casein composition contains casein, preferably selected from beta casein, alpha-S1 casein, alpha-S2 casein and mixture of these caseins, notably selected from beta casein, alpha-S1 casein and mixture of these caseins. As indicated, the microorganisms are transformed with a nucleic acid coding for alpha-casein and another nucleic acid coding for beta casein, wherein the two genes are under the control of a lactose inducible promoter.

It is preferred when the microorganisms are not transformed with a nucleic acid coding for kappa-casein. Consequently, it is preferred when no kappa casein is produced by the microorganisms, and when no kappa casein is present in the various compositions and solutions.

In the method herein disclosed, the lactoserum serves as an important source of carbon needed for the growth of the microorganisms,

In one embodiment, culture of the microorganisms is initiated in presence of a sugar other than lactose, as described above, in particular in the presence of glucose. When bacteria, in particular *E. coli* bacteria are used, as the microorganisms, glucose is present in an amount sufficient to induce CCR (carbon catabolic repression).

In such occurrence, bacteria are using glucose as the source of carbon and lactose metabolism is repressed. It is reminded that lactose metabolism involves the cleavage of one molecule of lactose to one molecule of glucose and one molecule of galactose. Such metabolism is repressed in the presence of glucose, as the carbon source in the culture medium, as the catabolites produced by the breakdown of glucose prevent the activation of the production of enzymes needed for lactose metabolism. However, once these enzymes are activated, repression no longer occurs (Görke and Stülke Nat Rev Microbiol 6, 613-624 (2008); Postma et al. Microbiol Rev. 1993 Sep;57(3):543-94).

In particular, it is preferred when glucose (or a sugar other than lactose) is used at the start of the culture, and during the early exponential phase of growth as it will ensure proper exponential growth of the bacteria.

At a later point of the exponential growth, or at the beginning of the stationary growth, lactoserum shall be used as a source of lactose for inducing expression of the caseins (if a lactose inducible system is used), or as a feedstock for stationary growth of the proteins.

In order to to trigger the switch to the lactose metabolism and lactose consumption (stop CCR), the amount of glucose is limited (glucose could even be depleted from the culture medium). Once this switch has occurred, it is possible to add glucose again in the culture medium or another sugar such as saccharose, fructose, galactose, notable in the form of molasse in the culture medium to complete the sugar supply.

In one embodiment, lactoserum is absent from the culture medium at the beginning of cultivation and is added, when the amount of the glucose is limited and when the switch to lactose consumption (and induction of protein expression) is desired (during exponential phase, or at start of stationary phase).

In another embodiment, lactoserum is added, with glucose, at the beginning of cultivation, and during the beginning of the exponential phase but it is not consumed as the concentration of glucose is maintained at a level high enough to induce CCR. Consequently, the switch to lactose consumption is achieved by reducing the glucose feed, for a given period of time (30 minutes may be enough), bacteria then using the lactose present in the lactoserum. Glucose may then be fed again, a low amount, so that there is no accumulation of glucose in the medium (this can be verified by sampling the medium and measuring glucose concentration). Generally, stopping glucose feed to suppress CCR and later feeding again with glucose would not lead to a reappearance of CCR.

Thus, lactoserum is present in the culture medium when the microorganisms are in the late exponential phase and/or stationary phase. In one embodiment, lactoserum is present in the culture medium when the microorganisms are in the exponential phase. In another embodiment, lactoserum is absent of the culture medium when the microorganisms are in the exponential phase.
it is also possible to use other sugars than glucose for starting the culture such as glycerol, as described by others (Viitanen et al. 2003, Cheese whey-induced high-cell-density production of recombinant proteins in Escherichia coli, Microbial Cell Factories 2:2; de Diviitis et al. (2023) Cheese-whey permeate improves the fitness. Biotechnology for Biofuels and Bioproducts, volume 16:30) to add lactoserum later (notably to induce expression of caseins) at an appropriate point of culture. Choice of the sugars and specific operating procedures to add lactoserum can easily be designed by one of skill in the art.

In one embodiment, the culture of the microorganisms is performed in a fed-batch reactor, by providing feed at periodic intervals. In another embodiment, the culture is performed in a continuous mode with continuous feed and withdrawal of product.

In another embodiment, the culture is performed in batch mode.

It is reminded that, during fed-batch fermentation, there is controlled addition of substrate and supplements into the fermentation medium, in contrast with batch mode, where all substrates are present in the bioreactor at the beginning of the process.

The "feed flow" describes the amount of culture medium, preferably comprising the microbial or bacterial host cell, that is transferred to the production reactor in a distinct period of time and may be expressed as volume per time, e.g. liters per minute. The feed flow may be regulated e.g. by an adjustable or controllable pump.

It is preferred when the concentration of the lactose in the culture medium is higher than 1%, and preferably between 1% and 5% (w/v, for 1g/100mL to 5g/100mL), when the switch to lactose consumption occurs.

In one embodiment, the lactoserum is raw or whole lactoserum, i.e. the liquid obtained from curdling or straining of milk. It contains some whey proteins.

In another embodiment, the lactoserum is permeate lactoserum, obtained by ultrafiltration of whole lactoserum, removing the lactoserum's serum proteins.

In one embodiment, one uses acid lactoserum (or derived permeate), obtained by coagulating milk through acidification with lactic acid bacteria, in this embodiment, a base is added to the culture medium to maintain the pH of the solution containing the microorganisms around pH 7.

In another embodiment one uses sweet lactoserum.

In one embodiment, the composition containing casein and other proteins (as well as potentially microorganisms, obtained from the culture of the microorganisms) is thus flown from the first container via a device containing a pipe or a tube, such tubular exchanger having the functions of both displacing the composition and heating it. There is thus a heat transfer (or heat exchange) during the passing of the composition within the pipe (corresponding to the heating step iv), so as to heat the composition, such transfer being performed on at least a fraction of the pipe. Such heating precipitates other proteins present in solution, while the casein remains in the soluble fraction.

The soluble fraction may be recovered by performing a centrifugation of the liquid composition flowing from the pipe.

The concentration of casein, and/or the ration of casein versus other proteins is higher in the recovered soluble fraction than in the starting composition, the casein thereby being enriched in this soluble recovered fraction.

As indicated, the composition of i) contains a soluble fraction and an insoluble fraction. The soluble fraction refers to a fraction that is not pelleted upon centrifugation. The insoluble fraction is the pellet obtained after centrifugation. Centrifugation may be performed at about 3000 g for 20-30 minutes. At industrial scale, continuous flow centrifugation, or another method such as filtration may be used to recover the soluble fraction. In the case of filtration, membranes of different types can be used.

The desired technical effect is obtained by heating during the passing in the tube, and preferably in the absence of organic solvents, or of addition thereof.

The device may contain, before the charcoal zone, a zone (such as a chamber or a length of a pipe) for pre-heating the composition before flowing within the pipe at the desired temperature. For instance, when the composition is at room temperature, the pre-heating may raise the composition temperature at about 80°C. The device may also present, after the heating pipe zone, a zone for cooling the composition, at temperature below 100°C (preferably about 60-80°C at most) for being able to recover the soluble fraction.

The ratio of casein is increased in the soluble fraction, when the relative amount of casein increases as compared to the total amount of proteins.

The other proteins are non-casein proteins. heating thus makes it possible to obtain a composition enriched in casein. Enrichment of casein in the composition refers to an increase of casein in the soluble fraction as compared to the amount present in the soluble fraction of the starting composition before flow through the pipe.

In one embodiment, the microorganism composition is washed to remove the culture medium before heating in iv).

As an illustration, it is possible to perform centrifugation of the microorganism cells (preferably bacteria) after culture, washing of the pellet, and resuspension in an appropriate liquid or fluid (appropriate buffer and more preferably in water, more preferably in the absence of organic solvents). The buffer is at a pH that is not acidic (equal or above 6.5), preferably close to neutral (between 6.5 and 7.5), or basic (preferably below 9). Indeed, caseins can precipitate at acidic pH. It is also preferred when the buffer is not ionic, or with a low ionic strength.

The resuspended composition is thus submitted to the heating step of iv.

In one embodiment, the microorganisms of the microorganism composition have been lysed to obtain a liquid composition before iv), wherein the liquid composition is in a first container. In another embodiment, the microorganisms in the microorganism composition are not lysed and heating in iv) lyses the microorganisms.

Lysis of the microorganisms may be performed, using any method known in the art, preferably using a chemical (such as detergent) or enzymatic lysis using for instance lysozyme or proteinase K, or mechanically, using a French press.

It is possible to optionally perform one further step chosen among addition of activated charcoal, chemical resins, membrane filtration (ultrafiltration, nanofiltration, or reverse osmosis), centrifugation, chromatography or precipitation of casein, to the soluble fraction recovered in v). The soluble fraction may notably be recovered by performing a centrifugation of the liquid composition flowing from the pipe, using regular or disc stack centrifuges Alternatively, it can be obtained by microfiltration or diafiltration.

Activated charcoal can be added to the soluble fraction and stirring is performed. Activated charcoal can be used to adsorb impurities (in particular organic impurities or chlorine) present in the soluble fraction, and that were not removed when recovering the soluble fraction in iii). Stirring with activated charcoal is preferably done, and the activated charcoal is removed before another method (such as the ones disclosed below) is performed on the soluble fraction.

Chromatography methods are widely used for the purification methods, and include notably affinity chromatography, ion exchange chromatography, hydrophobic interaction chromatography and others.

Membrane filtration, including notably ultrafiltration and nanofiltration is commonly used in protein purification as well (Saxena et al. (2009) Membrane-based techniques for the separation and purification of proteins: An overview. Advances in colloids and Interface Science Volume 145, pages 1-22), and membrane filtration techniques are widely used in the dairy industry. Interestingly, they can be used to separate the different caseins from each other (see above).

Finally, the specific properties of caseins (Post et al. (2012) Effect of temperature and pH on the solubility of caseins: Environmental influences on the dissociation of caseins. J. Dairy Sci. Volume 95 : pages 1603-1616), and notably, their propensity to precipitate in acidic conditions can be used for further purification.

In a preferred embodiment, further processing said soluble fraction is done by precipitation of caseins in acidic conditions. After precipitation, caseins can be resuspended, and using an appropriate basic buffer, they can also be resolubilized (Post et al. (2012) Effect of temperature and pH on the solubility of caseins: Environmental influences on the dissociation of caseins. J. Dairy Sci. Volume 95 : pages 1603-1616). In a preferred embodiment, further processing of said soluble fraction is done by precipitation of caseins at pH ranging from pH=4 to pH=5. In a more preferred embodiment, precipitation of caseins is conducted at pH of about 4.6. Various acids can be used including lactic acid, hydrochloric acid, and sulfuric acid.

Casein can then be recovered by methods such as centrifugation, or tangential filtration. A washing step can be added, with water adjusted at the pH used for precipitation. Separation of the washed casein can be made using the method as cited above.

Precipitation in acidic conditions is particularly interesting when the casein is isolated from a culture of microorganisms, as it makes it possible to remove or degrade any nucleic acid that may be present in the casein composition. Presence of DNA or RNA of the microorganisms may prove to be detrimental (at least from a regulatory point of view) when the casein is to be used for obtaining edible compositions intended to be ingested by human beings (such as cheese substitutes). pH is in the range of, and at about 4.6, and temperature ranging from 80°C to 140°C and times may vary between a few minutes and 2 hours.

Alternatively, recombinant DNA can be eliminated using nucleases such as DNAses that can be eliminated by heating.

When using microorganisms such as Gram- bacteria, one also could add other steps for elimination of lipopolysaccharides (LPS), and treatment at high temperature in acidic or basic conditions, the use of activated charcoal at high temperature, or filtration methods (10-20kDa separation, or various extraction meythods could be used).

pH can then be neutralized to obtain caseinates. For this, various basic coumpounds can be added such as liquid or gaseous ammonia, sodium hydroxide, potassium hydroxide or calcium hydroxide. Targeted pH can range between 6.5 and 9/. The kinetic of pH adjustment can continuous, or following a series of plateau, in order to achieve a stable target pH. before drying, caseins can be cocenctrated by filtration or evaporation. Caseins can then be dessicated by spray drying, flash drying, or other methods known in the field.

In a preferred embodiment, pH is adjusted using sodium hydroxide. In another embodiment, pH is adjusted using potassium hydroxide. In another embodiment, pH is adjusted using calcium hydroxide.

In a specific embodiment, the casein composition is desiccated. When obtained from a bacterial culture, such composition shall contain about 15-30% casein (w/w) and carbohydrates in the desiccated composition.

It is preferred when a little water is left, to favor future rehydration. Consequently, desiccation is to be understood as reducing the amount of water. In some embodiments, the amount of water is about 50% or less (w/w).

The insoluble fraction, containing most of the biomass produced during fermentation, can be separately treated and valorized. Such biomass can be used in feed or food applications, after using the same treatment as for caseins to remove recombinant DNA: pH is in the range of 4.6, with temperature ranging from 80°C to 140°C, and time may vary between a few minutes and 2 hours; alternatively, recombinant DNA can be eliminated using nucleases such as DNAses that can be eliminated by heating.

The composition of ii) is thus heated when flowing through the pipe, at a temperature comprised higher than 75°C. More preferably, the temperature is higher than about 80°C, more preferably higher than about 85°C, more preferably higher than about 90°C, more preferably higher than about 95°C, most preferably higher than about 100°C, or higher than 100°C. It may be advisable to use temperatures equal or higher than 105°C, or equal or higher than 110°C. It is possible to use temperatures as high as 140°C or even 150°C. It is preferred to heat the composition for 1 minute at most. In this case, an adequate temperature is preferably between 95°C and 115°C, preferably between 100°C and 115°C (limits included). A temperature higher than about 95°C is acceptable. A temperature of about 110°C would be appropriate. Such temperatures make it possible to obtain the technical and functional effects of removal of the other proteins, therefore reducing their amount and/or moving the casein from the insoluble to the soluble fraction, and while heating for a short duration (at most five minutes, preferably at most two minutes, but rather 90 seconds or less, or 75 seconds or less, more preferably 60 seconds or less, or even 45 seconds or less). The examples show that durations of 30 seconds or even 2 seconds can be applied. It is however preferred to heat the composition flowing within the pipe for at least 5 seconds or at least 10 seconds.

Pressure within the pipe is generally higher than 3 bars, more preferably 4 bars or more, more preferably 4.5 bars or more. It is generally 7 bars of less, preferably 6.5 bars or less, preferably 6 bars or less. A pressure between 4.5 and 5.5 bars (limits included) is well adapted.

The term "about", when referring to a measurable value, is meant to encompass variations of ±3% from the specified value, as such variations are appropriate to perform the disclosed methods.

It is advisable to pre-heat the composition arising from the first container before performing the heating step within the pipe. This is performed in a chamber out of the first container or in a pipe that conducts the composition from the first container to the pipe where it is heated at the desired temperature for the desired amount of time. The pre-heat temperature may be 20°C or 30°C less than the targeted heating temperature. Pre-heating between 75°C and 90°C (such as at 80°C) is well adapted.

In order to obtain the desired temperature within the pipe, and especially when heating by heat-exchange is performed, it is heated at a temperature higher than such desired temperature (generally about 5°C higher) as this takes into account the temperature gradient from the outside of the pipe to the center of the pipe. Heating can be performed by any of the following methods:

Direct steam injection: injecting steam directly into the pipes to heat them. The steam is generated in a boiler and directed into the pipes, where it transfers its heat to the pipe walls, heating the product flowing through.

Indirect heating by heat exchange: double-walled pipes are used. The composition flows through the inner tube, while steam circulates in the space between the two walls. The heat from the steam transfers through the pipe wall, heating the product.

Electric heating: heating elements are built into the pipes to heat them, electricity being used to generate heat directly in the pipe walls.

Induction beam heating: This method uses electromagnetic induction to heat the pipes, by Joule effect, by use of an alternating electric current induced in the pipe walls with the aid of a magnetic field.

In some embodiments, the composition is circulated within the pipe with a flow rate comprised between 15 and 30 L/h.

The composition is generally cooled and delivered to a second container after flowing. In some embodiments, the soluble fraction, that has been recovered by centrifugation and/or filtration after flowing and heating is delivered to the second container. Further purification or concentration of casein (for instance by ways of evaporation) and/or of quantification of the casein in the soluble fraction can then be performed.

Such method makes it possible to obtain a casein composition where the caseins represent at least 60 %, preferably at least 62 %, preferably at least 65 %, preferably at least 67 %, preferably at least 70 % of the total proteins of the soluble fraction. In particular, the ratio of casein as compared to the total proteins is increased in the soluble fraction as compared to the ratio in the microorganism composition.

In summary, the methods herein disclosed thus make it possible to obtain a casein composition with a low environmental footprint. Such a casein composition susceptible to be obtained (or obtained) by a method herein disclosed is a further subject of the invention. Such composition can be characterized as defined above.

In milk, the casein is in the micellar form found in milk, wherein micelles include alpha-S1, alpha-S2, beta and kappa caseins assembled in the same particules. In the context of the present invention, casein may not be assembled in the micellar form. However, this doesn't prevent ability to obtain a curd from the casein composition, by addition of an appropriate curdling agent.

Indeed, as developed in the examples, such casein composition can be used for producing cheese substitutes, and in particular animal-free cheese substitutes, *i.e.* cheese substitutes that don't contain any products of animal origin (in particular when the casein has been isolated from bacterial or yeast culture).

In summary, simple and short (less than 5 minutes, or preferably less than 2 minutes) heating of a composition containing casein and other proteins through a pipe makes it possible to enrich the composition in casein by reducing the amount of most of the other proteins in the soluble fraction. Heating also makes it possible to solubilize casein found in the insoluble fraction of bacterial cultures. The obtained casein, although not in the optimal condition for forming micelles, can appropriately curd upon the action of a curdling agent. Such findings were unexpected.

In particular, as shown in WO2022253816, curdling can be achieved with the recombinant caseins made in bacteria, and in the absence of kappa caseins, even though they don't possess post-translation modifications (such as phosphorylations) and the fact that kappa caseins are known to play an important role in the formation of dairy casein micelles.

In one embodiment is disclosed a method for obtaining a dairy substitute, comprising performing the method herein disclosed, allowing to obtain a casein composition, mixing the casein composition with at least one other ingredient comprising at least one component selected from the group consisting of proteins, water, calcium, lipids, and carbohydrate to obtain a liquid pre-curd composition (LpCC), and further processing the liquid pre-curd composition (LpCC) to obtain a dairy substitute. Further processing the LpCC notably includes providing a curdling agent to the LpCC to obtain a curd. Such curd can then be drained (and the obtained lactoserum may be used again in methods herein disclosed, therefore providing circular use), molded and aged to obtain a cheese substitute. The curd can be obtained and processed with lactic bacteria (*Streptococcus thermophilus* and *Lactobacillus bulgaricus*) to obtain yogurt substitutes.

Such embodiment is disclosed notably in WO2022058573 and WO2022253816.

As indicated, said curdling agent is preferably not rennet, but is an acidifying agent, notably a lactic bacteria, or lactic, citric or acetic acid. The casein composition may contain only alpha-S1 casein (as the casein protein), or only alpha-S2 casein (as the casein protein), or only beta casein (as the casein protein), or only alpha-S1 and beta casein (as the casein proteins), or only alpha-S2 and beta casein (as the casein proteins), or only alpha-S1 and alpha-S2 casein (as the casein proteins), or alpha-S1, alpha-S2 and beta casein.

The curd may be processed to obtain an edible composition, notably a cheese substitute, as disclosed in WO2022058573 and WO2022253816. These two documents are herein incorporated by reference, in particular the parts describing the obtention of a LpCC or of an edible composition, starting from a composition containing caseins, notably produced in bacteria and/or without kappa casein, in particular parts pertaining to obtaining a cheese substitute having the essential features of fresh cheese and a cheese substitute having the essential features of soft cheese or semi-soft cheese. The parts of these documents and in particular of WO2022253816, pertaining to calcium and other salts, lipids, emulsifying and gelling agents, carbohydrates, vitamins, curdling agents, ferments, moisture, and processing of the curd are also incorporated by reference.

The teachings pertaining to the composition of the LpCC, depending on the type of cheese substitute that is desired, are also incorporated by reference, as well as the amount of gelling agent to add (notably pages 24 to 28 of WO2022253816).

It is reminded that a gelling agent (in particular agar-agar) can be added in with the other ingredients before curdling or after curdling.

It is preferred when the other ingredients added in b) are all of non-animal origin.

The ingredients added in ii) comprise
i. Optionally proteins other than caseins
ii. Lipids
iii. Water, and
iv. Carbohydrates; in preferred embodiments, the carbohydrates do not comprise lactose, in order for the edible composition to be more acceptable to lactose-intolerant customers.

The choice and amount of the ingredients is adjusted so that the composition of the LpCC is adapted to the eventual composition desired for the edible composition, taking into account the loss of water due to curdling and aging (which can be modulated by one of skill in the art by modifying the duration and conditions of maturation).

In particular, processing of the LpCC comprises
vi. adding at least one curdling agent to the liquid composition so as to obtain a curd, and
vii. further processing said curd in order to obtain a dairy substitute, wherein the dairy substitute is a cheese substitute or a yoghourt substitute.

Such further step vi) may comprise acidic precipitation of casein. In particular, acidic precipitation is performed at at a pH between 4 and 5 (notably at pH=4.6), and preferably at about 90°C. Multiple other steps of purification can be performed.

Further processing of the curd may include the steps of
a) mixing a structuring agent with the curd, wherein the structuring agent is selected in the group consisting of gelling agents, texturing agents, emulsifying agents and mixtures thereof, to obtain a supplemented curd, and
b) molding and draining the supplemented curd.

One can also cite a method comprising
a) Providing a casein composition, comprising a recombinant casein as herein disclosed
b) Mixing said casein composition with at least one other ingredient comprising at least one component selected from the group consisting of water, calcium, lipids, and carbohydrate, so as to obtain a liquid pre-curd composition (LpCC),
c) Adding at least one curdling agent to the liquid pre-curd composition so as to obtain a curd
d) Further processing the curd (in particular by draining) to obtain an edible composition,
wherein a gelling agent (notably agar-agar) is added in b) and/or in d).

In one embodiment, the gelling agent is added in b).

In another embodiment, the gelling agent is added in d).

In another embodiment, the gelling agent is added in b) and in d).

In this embodiment, it is preferred when the edible composition
i. has a protein content comprised between 2 and 15% (weight content)
ii. has a moisture content superior to 80%, on a fat-free basis.

This embodiment is thus well adapted for production of a fresh cheese substitute. Further processing the curd may further include molding the drained curd, and/or aging the dried curd.

The environmental footprint of dairy substitutes, or any other dairy substitutes made with caseins obtained by the methods herein described, can be significantly reduced for at least one of the criteria listed above, as compared with a dairy substitute obtained by the same method, but wherein lactoserum is not added to produce casein by fermentation, and wherein the lactose from lactoserum is replaced with an equivalent mass of glucose, sucrose or sucrose molasses. In particular, the environmental foot print is reduced of at least 5%, or at least 10%, or at least 20 %, or at least 25 %, or at least 30 %.

### DESCRIPTION OF THE FIGURES

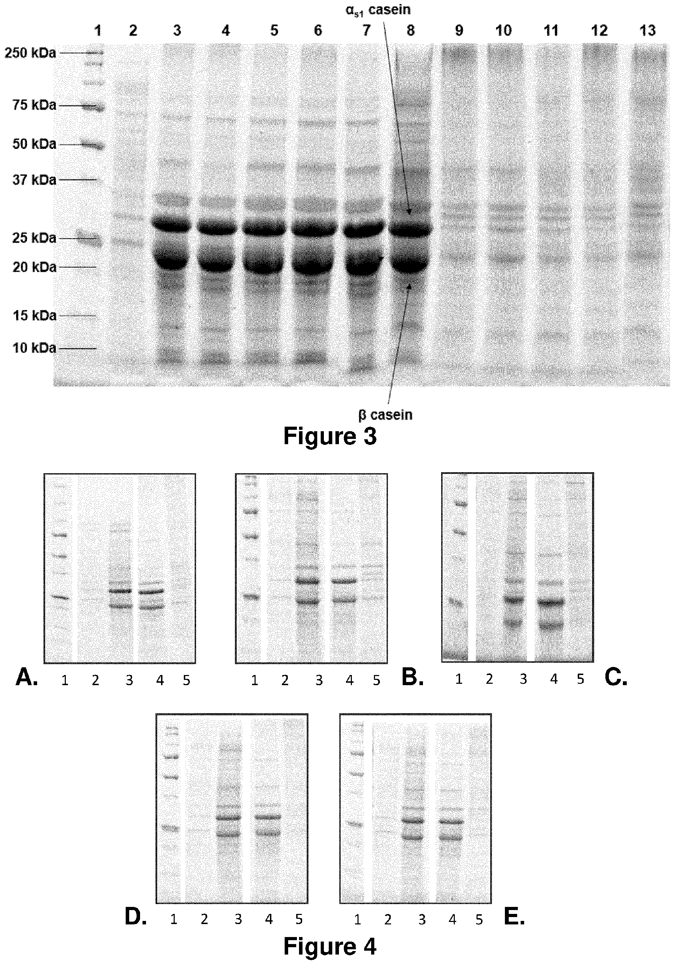

**Figure 1****:** Structure of plasmid expressing beta and alpha-S1 caseins.
**Figure 2****:** Monitoring of the supply pressure and of the temperature's product during the heating step and at the exit of the pasteurization chamber (tubular heat exchanger). Plain line: heating temperature of the product. Small dotted line: product's temperature at the exit of the pasteurization chamber. Large dotted line: supply pressure.
   A. Trial 1: Holding time : 110°C - Heating time: 75 seconds
   B. Trial 2. Holding time : 110°C - Heating time: 30 seconds
   C. Trial 3. Holding time : 120°C - Heating time : 30 seconds
   D. Trial 4 : Holding time : 140°C - Heating time : 30 seconds
   E. Trial 5 : Holding time : 140°C - Heating time : 2 seconds
**Figure 3****:** SDS-PAGE analysis of the αₛ₁, and β caseins in the lysate, before and after thermal treatment, in soluble and insoluble fractions. **Lane 1:** Size marker, Precision Plus Protein^{™} Unstained Standards (Bio-Rad, 1610363EDU) - **Lane 2:** Cell suspension before thermal treatment, soluble fraction) - **Lane 3:** Lysate after thermal treatment (Trial 1), soluble fraction - **Lane 4:** Lysate after thermal treatment (Trial 2), soluble fraction - **Lane 5:** Lysate after thermal treatment (Trial 3), soluble fraction - **Lane 6:** Lysate after thermal treatment (Trial 4), soluble fraction - **Lane 7:** Lysate after thermal treatment (Trial 5), soluble fraction - **Lane 8:** Cell suspension before thermal treatment, insoluble fraction - **Lane 9:** Lysate after thermal treatment (Trial 1), insoluble fraction - **Lane 10:** Lysate after thermal treatment (Trial 2), insoluble fraction - **Lane 11:** Lysate after thermal treatment (Trial 3), insoluble fraction - **Lane 12:** Lysate after thermal treatment (Trial 4), insoluble fraction - **Lane 13:** Lysate after thermal treatment (Trial 5), insoluble fraction.
**Figure 4****:** SDS-PAGE analysis of the αₛ₁, and β caseins in the lysate, before and after thermal treatment, in soluble and insoluble fractions. Analysis of 3-fold diluted samples. For each panel, **Lane 1:** Size marker, Precision Plus Protein^{™} Unstained Standards (Bio-Rad, 1610363EDU) - **Lane 2:** Cell suspension before thermal treatment, soluble fraction - **Lane 3:** Cell suspension before thermal treatment, insoluble fraction - **Lane 4:** Lysate after thermal treatment, soluble fraction - **Lane 5:** Lysate after thermal treatment, insoluble fraction. Panel A: 110°C, 75 seconds; B: 110°C, 30 seconds; C: 120°C, 30 seconds; D: 140°C, 30 seconds; E: 140°C, 2 seconds.

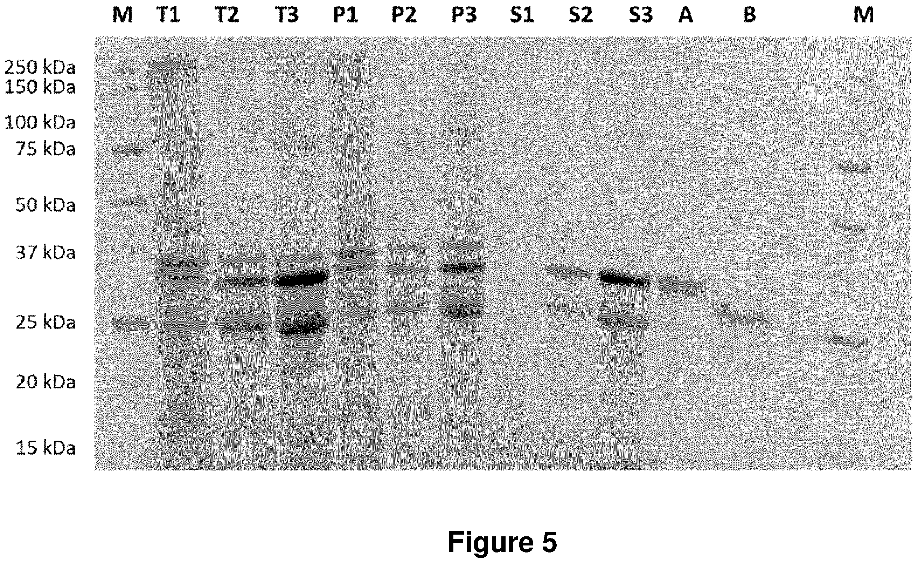

**Figure 5****: Analysis of casein production by SDS-PAGE in various induction conditions** Samples were prepared as described in Example X. **T1, T2, T3:** total lysates from uninduced culture in glucose **(T1),** culture with glucose induced with IPTG **(T2)** culture with glucose and lactoserum (**T3**, as described in example X). **P1, P2, P3:** insoluble faction (pellet) of lysates from uninduced culture in glucose **(P1),** culture with glucose induced with IPTG **(P2)** culture with glucose and lactoserum **(P3). S1, S2, S3:** soluble fractions (supernatant) of lysates from uninduced culture in glucose **(S1),** culture with glucose induced with IPTG **(S2)** culture with glucose and lactoserum (**S3)**. **A:** purified Alpha S1 and Alpha S21 caseins from Sigma 1g/L. **A:** purified beta casein from Sigma 1g/L. **M:** molecular weight marker. Band sizes were indicated left of the gel.

### EXAMPLES

### Example 1: Production of alpha-S and beta caseins

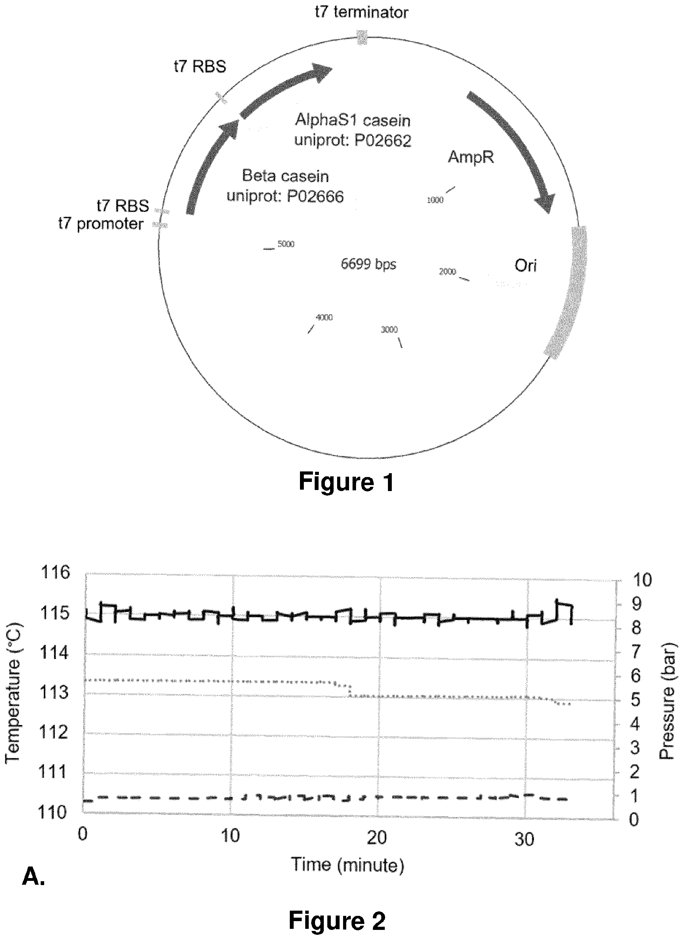

Synthetic genes coding for alpha-S1 and beta casein (related to natural genes P02662, and P02666, respectively), were modified, in order to remove the signal peptide, and the sequence of the new synthetic open reading frames (SEQ ID NO: 2 and SEQ ID NO: 4) are shown in Table 2, last column. They were cloned into pET25b+ (Figure 1) and the resulting plasmid was transformed into BL21(DE3) strains (Novagene). Individual transformed clones were isolated, and for each synthetic gene, one clone was used to inoculate LB medium.

**Table 2: Sequences of natural caseins (precursors) and of the related recombinant proteins. In natural caseins, signal peptides are indicated in bold. In recombinant caseins, the methionine resulting from cloning into the pET expression system is indicated in bold.**

| Name and Uniprot reference of protein precursor | sequence of casein precursor | sequence of recombinant casein |
|---|---|---|
| Alpha-S1 casein P02662 | MKLLILTCLVAVALARPKHPIK HQGLPQEVLNENLLRFFVAPFP EVFGKEKVNELSKDIGSESTED QAMEDIKQMEAESISSSEEIVP NSVEQKHIQKEDVPSERYLGYL EQLLRLKKYKVPQLEIVPNSAE ERLHSMKEGIHAQQKEPMIGV NQELAYFYPELFRQFYQLDAY PSGAWYYVPLGTQYTDAPSFS DIPNPIGSENSEKTTMPLW (SEQ ID NO: 1) | MRPKHPIKHQGLPQEVLNENLLR FFVAPFPEVFGKEKVNELSKDIG SESTEDQAMEDIKQMEAESISSS EEIVPNSVEQKHIQKEDVPSERY LGYLEQLLRLKKYKVPQLEIVPNS AEERLHSMKEGIHAQQKEPMIGV NQELAYFYPELFRQFYQLDAYPS GAWYYVPLGTQYTDAPSFSDIPN PIGSENSEKTTMPLW (SEQ ID NO: 2) |
| Beta casein P02666 | MKVLILACLVALALARELEELN VPGEIVESLSSSEESITRINKKIE KFQSEEQQQTEDELQDKIHPF AQTQSLVYPFPGPIPNSLPQNI PPLTQTPVVVPPFLQPEVMGV SKVKEAMAPKHKEMPFPKYPV EPFTESQSLTLTDVENLHLPLP LLQSWMHQPHQPLPPTVMFPP QSVLSLSQSKVLPVPQKAVPY PQRDMPIQAFLLYQEPVLGPV RGPFPIIV (SEQ ID NO: 3) | MRELEELNVPGEIVESLSSSEESI TRINKKIEKFQSEEQQQTEDELQ DKIHPFAQTQSLVYPFPGPIPNSL PQNIPPLTQTPVVVPPFLQPEVM GVSKVKEAMAPKHKEMPFPKYP VEPFTESQSLTLTDVENLHLPLPL LQSWMHQPHQPLPPTVMFPPQ SVLSLSQSKVLPVPQKAVPYPQR DMPIQAFLLYQEPVLGPVRGPFP IIV (SEQ ID NO: 4) |

In order to produce a batch of alpha-S1 and beta caseins, the resulting strain was cultivated as follow:
Five concentrated stocks of the strain of each 1 mL, stored in 10 % DMSO at -80°C, were thawed and used to inoculate five precultures of a volume of 1 L each, respectively (0.1 % *v*/*v* inoculation rate), in a revivification medium Y15 composed of yeast extract (15 g/L) and NaCl (5 g/L), and supplemented with 100 µg/mL of ampicillin. The five precultures were grown in 4 L or 5 L erlenmeyers at 30°C, for 9 hours, at 170 rpm (orbital diameter: 25 mm). After 9 hours, optical densities (60nm) in the range of 3 were achieved.

2.4 L of the first preculture were used to inoculate a volume of 120 L (2% *v*/*v* inoculation rate), in an optimized culture media supplemented with 100 µg/mL of ampicillin and with 15 g/L of glucose. Growth parameters (oxygen pressure, stirring, pressure, aeration rate and pH regulation) were optimized to achieve an optical density (600nm) in the range of 15.

90 L of this culture were used to inoculate a fermentor with an initial volume of 1500 L (6 % v/v inoculation rate), in an optimized culture media, supplemented with 100 µg/mL of ampicillin. Growth parameters (glucose feed, oxygen pressure, stirring, pressure, aeration rate and pH regulation) were optimized to achieve an optical density (600nm) in the range of 90. IPTG was added at a final concentration of 0,2mM when optical density reached 20. This resulted in around 2 tons of culture broth with 3,4% of dry biomass.

### Example 2: Thermal bacterial lysis by ultra-high temperature processing (UHT)

A protocol was set for extraction and solubilization of αₛ₁, and β caseins from cells at ultra-high temperature.

### Biomass concentration and washing

The culture medium obtained as described in Example 1 was concentrated using a self-cleaning disk-stack centrifuge (SSE20, GEA, centrifugal force : 20000 G, bowl rotation speed : 12000 rpm, bowl filling : 80% of the capacity, feed rate : 500 L/h, back pressure : 5 bars, temperature < 20 °C, settling time : 54 seconds for the first solid/liquid separation (biomass concentration) and 40 seconds for the three washing steps of the biomass). 298 kgs of concentrated cells were collected, while 1670 kgs of fermentation medium was discarded.

The concentrated biomass was then washed three times with osmosis water, using the same centrifuge. The concentrated biomass was mixed with water, until obtaining a homogeneous suspension (11.17 % of dry mass). The suspension was then separated by centrifugation, and the first fraction of washing water (800.4 kg) was eliminated. This washing procedure was repeated twice; to finally obtain a pellet of concentrated cells of 239.1kg. The color of the supernatant was less and less yellow, until obtaining a slightly colored yellow washing water at third wash.

### Thermal bacterial lysis

Washed concentrated biomass was then resuspended in 4,2 volumes of osmosis water, until having a homogeneous suspension. 60 L of this resulting suspension were stored during four days at +4°C, without any stirring. After four days, the pH of the suspension was 6.654 ± 0.057. The suspension was then divided in four fractions of 10 kgs, and one fraction of 8.15 kgs. The pH of each suspension was adjusted at 8.5, using a 2 M solution of sodium hydroxide (NaOH). pH and temperature of the different fractions collected before and after the pH adjustment are described in **Table 3**.

**Table 3: pH and temperature of the different fractions before and after pH adjustment to 8.5**

| **Trial** | **Product** | **pH** | **Temperature** |
|---|---|---|---|
| | Washed biomass | 6.620 | 7°C |
| 1 | After pH adjustment | 8.501 | 20°C |
| | Washed biomass | 6.700 | 10°C |
| 2 | After pH adjustment | 8.500 | 22°C |
| | Washed biomass | 6.700 | 18°C |
| 3 | After pH adjustment | 8.509 | 34°C |
| | Washed biomass | 6.680 | 15.6°C |
| 4 | After pH adjustment | 8.481 | 21°C |
| | Washed biomass | 6.570 | 12°C |
| 5 | After pH adjustment | 8.498 | 22°C |

The five fractions were then heated by monitoring a holding time at a given temperature in a pasteurization chamber), using a UHT Pasteurizer (Pasteurizer, OMVE, HTST/UHT System, HT220-DSI, initial temperature of the product: 20°C, configuration : tubular heat exchanger, flow rate : 16.66-27.2 L/h, holding time : 2-75 seconds, pre-heating temperature : 80°C, heating temperature : 113-145°C, temperature in the pasteurization chamber: 110-140°C, pre-cooling temperature : 80°C, cooling temperature : 40°C, pressure : 5 bars).

Five different conditions were tested on the five bacterial suspensions, in order to determine an optimal and fast thermal treatment, allowing efficient bacterial lysis, *E.coli* proteins precipitation and caseins solubilization. In each case, the applied flow rate, the holding time, the pre-heating and heating temperatures, and the pre-cooling and cooling temperatures are described in **Table 3**. The different set heating temperatures were slightly higher than the temperatures applied in the pasteurization chamber, to ensure that those last temperatures would be reached.
**Trial 1** : temperature = 110 °C, holding time = 75 seconds
**Trial 2** : temperature = 110°C, holding time = 30 seconds
**Trial 3** : temperature = 120 °C, holding time = 30 seconds
**Trial 4 :** temperature = 140 °C, holding time = 30 seconds
**Trial 5** : temperature = 140 °C, holding time = 2 seconds, at micro-pilot scale

The general conditions are described in detail in Table 4. All of these different conditions allow a total deactivation of the bacterial *E.coli* strain used in fermentation.

**Table 4: Experimental conditions of the five different trials of thermal treatments**

| **Trial** | **Parameters** |
|---|---|
| 1 | Product : 10 kgs |
| | Initial temperature of the product : 20°C |
| | Configuration : tubular heat exchanger |
| | Flow rate : 16.66 L/h |
| | Holding time : **75 seconds** |
| | Pre-heating temperature : 80°C |
| | Heating temperature : 115°C |
| | Temperature in the pasteurization chamber : **110°C** |
| | Pre-cooling temperature : 80°C |
| | Cooling temperature : 40°C |
| 2 | Product : 10 kgs |
| | Initial temperature of the product : 20°C |
| | Configuration : tubular heat exchanger |
| | Flow rate : 27.2 L/h |
| | Holding time : **30 seconds** |
| | Pre-heating temperature : 80°C |
| | Heating temperature : 113°C |
| | Temperature in the pasteurization chamber : **110°C** |
| | Pre-coolinq temperature : 80°C |
| | Cooling temperature : 40°C |
| 3 | Product : 10 kgs |
| | Initial temperature of the product : 20°C |
| | Configuration : tubular heat exchanger |
| | Flow rate : 27.2 L/h |
| | Holding time : **30 seconds** |
| | Pre-heating temperature : 80°C |
| | Heating temperature : 124°C |
| | Temperature in the pasteurization chamber : **120°C** |
| | Pre-cooling temperature : 80°C |
| | Cooling temperature : 40°C |
| 4 | Product : 10 kgs |
| | Initial temperature of the product : 20°C |
| | Configuration : tubular heat exchanger |
| | Flow rate : 27.2 L/h |
| | Holding time : **30 seconds** |
| | Pre-heating temperature : 80°C |
| | Heating temperature : 145°C |
| | Temperature in the pasteurization chamber : **140°C** |
| | Pre-cooling temperature : 80°C |
| | Cooling temperature : 40°C |
| 5 | Product : 8.5 kgs |
| | Initial temperature of the product : 20°C |
| | Configuration : tubular heat exchanger |
| | Flow rate : 20 L/h |
| | Holding time : **2 seconds** |
| | Pre-heating temperature : 80°C |
| | Heating temperature : 142°C |
| | Temperature in the pasteurization chamber : **140°C** |
| | Pre-cooling temperature : 80°C |
| | Cooling temperature : 40°C |

Product: amount of treated sample. Initial temperature of the product: initial temperature of the product to be treated. Configuration: tubular chamber (Pasteurization chamber) preceded by 2 heat exchangers (one heat exchanger where the outlet of the treatment heat up the product to be treated - one heat exchanger to complete the heat up prior the tubular chamber) and followed by 1 heat exchanger that cool down the treated product preliminary cool down. Flow rate: Flow rate of the product through the pasteurization chamber. Holding time: Time spent by the product in the pasteurization chamber. Pre-heating temperature: Temperature reached by the product at the outlet of the first heat exchanger. Heating temperature: Maximum temperature reached by the product at the inlet of the chamber. Temperature in the pasteurization chamber: Temperature of the product in the tubular chamber. Pre-cooling temperature: Temperature reached by the treated product at the coolest outlet of the first heat exchanger. Cooling temperature: Temperature of the treated product reached at the outlet of the final cooling heat exchanger.

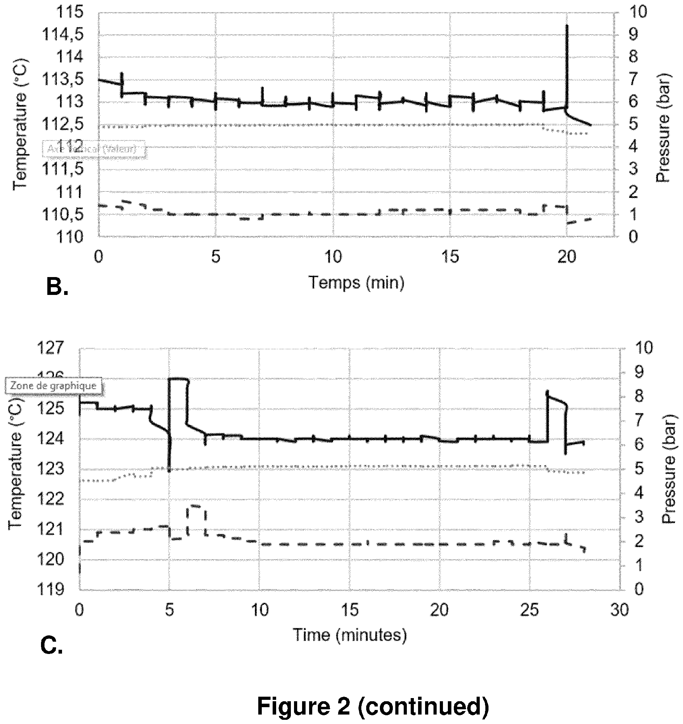

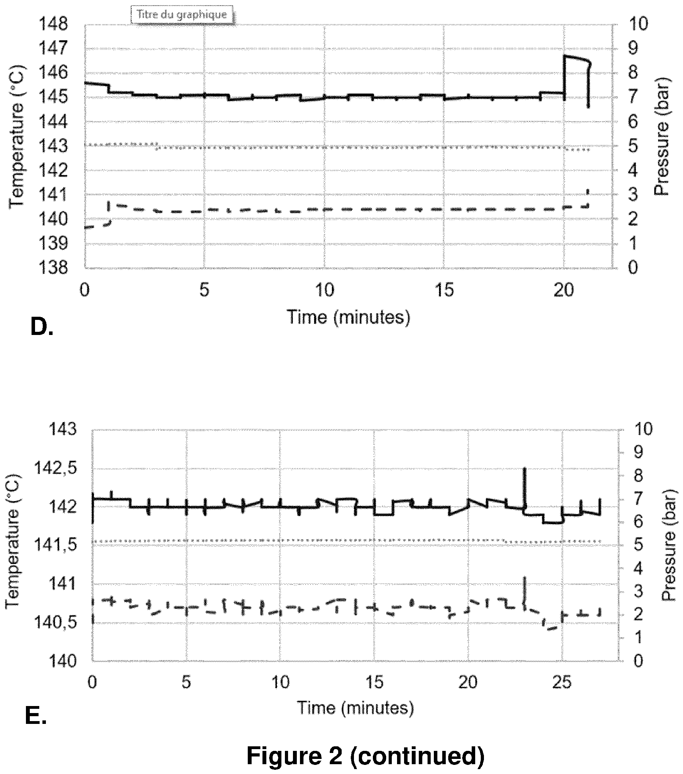

In each case, the temperature of the product was controlled during the heating step and at the exit of the pasteurization chamber, while the supply pressure was measured during all the process. The monitoring of these parameters is presented in Figures 2 to 6.

For trial 1 to trial 5, the weight of each fraction was measured, before and after the heating step, as well as the pH and the temperature. Moreover, the quantity of 2 M NaOH solution added to adjust the pH to 8.5 was also determined (**Table 5**).

**Table 5: Monitoring of the pH, the temperature, and the weight of each fraction collected in trial 1 to trial 5, as well as the weight of 2 M NaOH solution added.**

| **Trial** | **Product** | **pH** | **Temperature** | **Weight [kg]** |
|---|---|---|---|---|
| 1 | Washed biomass | 6.620 | 7°C | 10.00 |
| | After pH adjustment | 8.501 | 20°C | - |
| | 2 M NaOH solution added | - | - | 27.30 |
| | After thermal treatment | 7.640 | 32°C | 9.165 |
| 2 | Washed biomass | 6.700 | 10°C | 10.00 |
| | After pH adjustment | 8.500 | 22°C | - |
| | 2 M NaOH solution added | - | - | 25.80 |
| | After thermal treatment | 7.593 | 30°C | 9.110 |
| 3 | Washed biomass | 6.700 | 18°C | 10.00 |
| | After pH adjustment | 8.509 | 19°C | - |
| | 2 M NaOH solution added | - | - | 25.60 |
| | After thermal treatment | 7.640 | 34°C | 9.030 |
| 4 | Washed biomass | 6.680 | 15.6°C | 10.00 |
| | After pH adjustment | 8.481 | 21°C | - |
| | 2 M NaOH solution added | - | - | 27.74 |
| | After thermal treatment | 7.790 | 32°C | 8.900 |
| 5 | Washed biomass | 6.570 | 12°C | 8.15 |
| | After pH adjustment | 8.498 | 22°C | - |
| | 2 M NaOH solution added | - | - | 22.18 |
| | After thermal treatment | 7.650 | 34°C | 7.115 |

After the different applied thermal treatments, pH of each fraction decreased, from 7.790 to 7.593, *i.e.* a decrease of 0.71 to 0.90 pH unit. From 10 kgs of product, between 8.9 kgs to 9.165 kgs were recovered, in trial 1 to trial 4. For the last trial, a fraction of 7.115 kg was obtained from an initial weight of 8.15 kgs.

In a view to investigate the efficiency of these five different heat treatments, samples were collected before and after each trial and analyzed by sodium dodecyl sulfate-polyacrylamide gel electrophoresis (SDS-PAGE) on a polyacrylamide gel (4-20% Criterion^{™} TGX Stain-Free Protein Gel, Bio-Rad, 5678093) with Precision Plus Protein^{™} Unstained Standards (Bio-Rad, 1610363EDU) as ladder.

For each trial, 1 mL of bacterial suspension was taken before and after the applied heat treatment, and centrifuged (13000 rpm, 5 minutes, RT), to separate the soluble and insoluble fractions. The supernatant was separated from the pellet, and mixed in a *1:1* ratio with the 2X sample buffer, composed of 0,1 M Tris-HCI, *4% v:v SDS,* 0,2% *w:v* bromophenol blue and 20 % *v:v* glycerol. The cell pellet was mixed with 1 mL of a 2% *v:v* SDS solution until obtaining a homogeneous suspension, and mixed in the same ratio with the 2X sample buffer previously described. Each resulting solution was heated at 95°C for 5 minutes and loaded in the wells of a polyacrylamide gel, using a running buffer composed of Tris-HCI (25 mM), glycine (250 mM) and SDS (0,1% *v:v*). The gel was then visualized under UV light, in a Gel-Doc^{™} EZ Imager (Bio-Rad). Diluted samples (3-fold) were also used for better quantification. The ratio of casein content vs total protein content was determined *via* the analysis of each SDS PAGE gels using the software Imagelab, by comparing bands' intensity of caseins and bands' intensity of other proteins from *E. coli.*

Results are summarized in Figures 3 and 4, with undiluted (Figure 3) and diluted (Figure 4) samples. In all tested conditions for pasteurization, *E.coli* cells were efficiently lysed and a large majority of αₛ₁, and β caseins were solubilized: total amounts of caseins amounts were very similar before and after pasteurization, but these caseins were essentially in the insoluble fraction before pasteurization (Figure 3, lane 8) and in the soluble fraction after pasteurization (Figure 3, lanes 3-7), indicating the process yield was very high (at least >80%).

The proportion of casein vs. total protein in the soluble fraction of the lysate (Figure 4, lanes 4 in each panel) was significantly the same in the five trials, ranging from ranging from 75 % to 77 %. This ratio appeared to be significantly higher than in the insoluble faction of cells before lysis (Figure 4, lanes 3 in each panel), which displayed many additional proteins bands, as can be seen on Figure 4 (lanes 3 in each panel). This ratio was a fortiori higher than in the cell composition (insoluble + soluble) before lysis, showing that the lysate soluble fraction was enriched in caseins as compared with the initial composition.

In addition, the average αₛ₁, casein/β casein ratio was also very similar between the five trials, ranging from 47% to 53%, similar to the initial composition in the cell before treatment, showing that in the condition we used, the was no bias regarding the purification of αₛ₁, casein vs. β casein.

### Example 3: Thermal lysis of bacteria

As described in WO2022253816, synthetic genes coding for alpha-S1, alpha-S2 and beta casein (related to natural genes P02662, P02663 and P02666, respectively), were modified, in order to remove the signal peptide. They were cloned into plasmids and the resulting plasmids were transformed into BL21(DE3) strains. Individual transformed clones were isolated, and for each synthetic gene, one clone was used to inoculate LB medium.

Cells were lysed using two different protocols, and total extracts as well as samples from soluble and insoluble fractions were analyzed by SDS page. Caseins were monitored in the soluble and insoluble fractions of cell extracts using two different protocols. Cells transformed with empty vector were used as control.

### Protocol 1:

Cell pellets obtained from a 100 mL culture were lysed by resuspension in lysis buffer (50 mM Tris HCI pH 7.5, 1 mg/mL lysozyme, 0,03 mg/mL Dnase). The suspension was incubated on ice for 30 min and then sonicated for 10 seconds (10% amplitude, Q Sonica XL-2000). 10 µL of the total fraction, were taken and kept for SDS-PAGE analysis. The soluble and insoluble fraction were separated by centrifugation at 3220 g and 4°C for 20 min. The supernatant was recovered and supplemented with 10% glycerol for preservation. The pellet was further resuspended in Tris 50 mM with SDS 2% and supplemented with 10% glycerol for preservation. 10 µL of each fraction were taken for SDS-PAGE.

### Protocol 2:

Cell pellets obtained from a 100 mL culture were lysed by resuspension in Bugbuster (Millipore) with 0,4% Lysonase (Millipore). The mix was incubated at room temperature for 5 min and then centrifuged at 3220 g and 4°C for 20 min. The supernatant was recovered and 10 µL of the supernatant were taken and reserved for SDS-PAGE analysis. The pellet was further resuspended in SDS 2% and 10 µL of the suspension, representing the insoluble fraction, were then taken and preserved for SDS-PAGE analysis.

Alpha-S1 casein was found in both the soluble (S) and insoluble (P) fractions, while alpha-S2 was found essentially in the insoluble fraction, and beta caseins largely (protocol 1) or totally (protocol 2) in the insoluble fraction, as often observed with overexpression of recombinant proteins in E. coli. This suggests that in E. coli, caseins may be present in inclusion bodies. The different quantitative outcomes can be due to different solubilities of beta casein or different stabilities of inclusion bodies depending on the protocol. Nevertheless, recovery of caseins from the soluble fraction of the extracts would result in loss of a large part or of all of the recombinant proteins using these protocols.

The impact of lysis by heating on caseins was tested with the recombinant strains described above. A clone transformed with empty vector was used as control.

Cultures were centrifuged and cell pellets were resuspended in 1 volume of sterile water, centrifuged and washed with another volume of water, and resuspended in 1 volume of water. 1 mL samples of this cell suspension were treated by heating at 95°C for 0 (non-heated), 10, 20, 30, 60, 90 and 120 minutes, resulting in cell lysis, and the soluble and insoluble fractions were separated by centrifugation. The insoluble fraction was resuspended in 1 mL of buffer (50 mM Tris HCI pH 7.5, 300 mM NaCl, 10 mM MgCl2, 2 mM DTT, 0.5% Triton and Sigmafast Protease Inhibitor (Sigma)) and 10 µL aliquots of both soluble and insoluble fractions were analyzed by SDS-PAGE. In such conditions a 10 µL sample of soluble fraction and a 10 µL sample of insoluble fraction represent about the same amount of total cell extract. With no heating, no cell lysis, or only residual cell lysis in water occurred. The pellet contains basically the entire cells, including the caseins, and this sample actually represents total cell extracts.

It was confirmed that lysis by temperature impacted the distribution of caseins in the soluble and insoluble fractions.

By (or before) 10 minutes of heating at 95°C, alpha-S1 casein was almost entirely found in the soluble fraction, whereas it was found in both the soluble and soluble fraction in similar amounts without heating.

Casein beta was progressively shifted to the soluble fraction, which contained large amount of this recombinant protein by (or before) 10 minutes of heating, and the major part by 30 minutes. Without heating, beta casein was found mostly or entirely in the insoluble fraction.

The distribution of casein apha-S2 (entirely in the insoluble fraction without heating) appeared to be less heat-sensitive, and it was still found in great part in the insoluble fraction by 120 minutes of heating. Nevertheless, it appeared in the soluble fraction by 20 minutes of heating at 95°C, and was slightly decreasing in the insoluble fraction over time.

Many other protein bands from the insoluble fraction were diminishing as well over time, but without being shifted into the soluble fraction. In the soluble fraction of the heated samples, only a few faint protein bands could be seen in addition to the casein bands.

These results indicate that lysis by heating is a good way to perform fast purification of caseins, and notably of alpha-S1 and beta casein.

### Example 4 : Production of recombinant casein in a 1L fermenter

Acid lactoserum was used as a feedstock, in conjunction with glucose. In order to generate acid lactoserum, semi-skimmed pasteurized milk was incubated with 0,1% of lactic bacteria (Choozit MBT LYO 20 DCU) at 34°C, for about 8 hours, until pH reached a value of about 4,6, to induce curdling. The curd was drained for 48 hours, to recover the lactoserum, and 0,77 kg of lactoserum was recovered for each liter of milk, with a pH of 4,6 and a lactose concentration of 51,3 g/L. This lactoserum was then filtered with a 0.22 mM filter and autoclaved. SDS-PAGE analysis indicted that no residual casein could be observed in this lactoserum.

A culture of recombinant strain described above was used to inoculate a 1L fermenter (Multifors 2 Microbila 1,4L TV, Infors), in a medium lactoserum and glucose, as carbohydrate sources.

A medium containing 50% of lactoserum was used, supplemented with 0.71g/L Na₂SO₄, 11,3 g/L (NH₄)₂SO₄, 0.85g/L KH₂PO₄, 1,11 g/L Na₂HPO₄, 4 g/L MgSO₄(7H₂O), 1,6 mL/L of 5% antifoam solution (Struktol), and trace elements (0,2% of a 500x composition described in Table 5). This medium was also supplemented with 100 µg/ml ampicilline and 2 g/L glucose. In this medium, lactose concentration was about 25,7g/L.

**Table 5 Composition of Trace element solution (500x)**

| **Ingredients** | |
|---|---|
| FeCl₃.6H₂O (g/L) | 13.5 |
| HCl 37% (mL/L) | 5 |
| CaCl₂.2H₂O (g/L) | 2.94 |
| MnCl₂.4H₂O (g/L) | 1.98 |
| ZnSO₄.7H₂O (g/L) | 2.875 |
| CuCl₂.2H₂O (g/L) | 0.342 |

0.5L of medium was inoculated, with a preculture of the recombinant clone, to reach an initial OD₆₀₀ of 0.25 . The fermentation was conducted in a fed-batch mode, at pH=7.2; T=37°C; pO₂=15%, with the culture being fed with a solution containing 600 g/L of D-Glucose. Ampicillin was added after 14 hours (100 µg/ml of culture).

Lactose is capable of inducing the expression of caseins from the pET vector in BL21. However, at the beginning of the run, glucose was capable of repressing lactose uptake, by carbon catabolic repression (Görke, B., Stülke, J. Carbon catabolite repression in bacteria: many ways to make the most out of nutrients. Nat Rev Microbiol6, 613-624 (2008); Postma PW, Lengeler JW, Jacobson GR. Phosphoenolpyruvate:carbohydrate phosphotransferase systems of bacteria. Microbiol Rev. 1993 Sep;57(3):543-94). Thus, at the beginning of the run, only glucose is consumed.

After about 15 hours, with OD₆₀₀=40, the glucose feed was interrupted for 30 min enabling the metabolic switch enabling lactose consumption and casein expression could be induced. After the 30 min, the glucose feed was reduced to enable culture growth without preventing lactose consumption. In this regard, the induction process was different from the one used by Viitanen et al. (Viitanen et al. 2003, Cheese whey-induced high-cell-density production of recombinant proteins in Escherichia coli, Microbial Cell Factories 2:2); in this study, induction was achieved by addition of lactoserum in a medium containing no glucose but supplemented in glycerol. The culture was stopped at T=21 h, with OD₆₀₀=100.7. Cells were harvested by centrifugation, at room temperature.

As a negative control, the same medium was used, without lactoserum (replaced with water). Thus, glucose is the only carbohydrate source, and no casein expression is expected. The culture was stopped at T=21 h, with OD₆₀₀=94.6. Cells were harvested by centrifugation, at room temperature. As a positive control for casein expression, we used the same medium without lactoserum (replaced with water), and induction was achieved by the addition of Isopropyl β-D-1-thiogalactopyranoside (IPTG), a lactose analogue, which inducing effect is not inhibited by glucose, at OD=40. The culture was stopped at T=21 h, with OD₆₀₀=80.0. Cells were harvested by centrifugation, at room temperature. Conditions are summarized in Table 6.

**Table 6. Summary of culture conditions, and induction mechanisms in the different conditions**

| | Glucose, non induced | Glucose, induced | Lactoserum and glucose, induced |
|---|---|---|---|
| lactoserum | - | - | + |
| Lactose | + | - | + (from lactoserum) |
| Glucose | 2g/L in the initial medium, and glucose feed | 2g/L in the initial medium, and glucose feed | 2g/L in the initial medium, and reduced glucose feed |
| Induction step | Casein expression remains repressed by glucose | IPTG addition. | Induction by lactose, upon glucose depletion when the glucose feed is interrupted |

For analysis, an aliquot of cells was centrifuged and resuspended in 1/2 volume of sterile water. 1 mL samples of this cell suspension were treated by heating at 95°C for 60 minutes, resulting as described previously (PCT/EP2022/064728) in cell lysis, casein solubilization, and degradation/and or precipitation of other proteins in the soluble factions and the soluble and insoluble fractions were separated by centrifugation. The insoluble fraction was resuspended in 1 mL of a 2% SDS solution and 10 µL aliquots of both soluble and insoluble fractions were analyzed by SDS-PAGE, together with total lysate..

Purified beta casein from milk (Sigma) was used as control, but as observed previously by others displayed a higher apparent molecular weight (Simons et al. Overproduction of bovine beta-casein in Escherichia coli and engineering of its main chymosin cleavage site (1993) Protein Engineering 7:763-770).

As shown on Figure 5, induction casein expression was efficiently induced both by IPTG in the presence of glucose and in the medium containing lactoserum. Casein production was very efficient with lactoserum. Recombinant casein production was estimated to be in the range of 2.5 g/L I glucose, and 5 g/L with lactoserum, twice more, and 1, times higher, when normalized to cell density (see OD₆₀₀ above).

The amount of consumed sugars was estimated in all samples. In the conditions used, E. coli BL21 is capable of glucose uptake and metabolization. At low glucose concentrations, it is also capable of lactose uptake, and hydrolysis into glucose and galactose; whereas galactose is very poorly consumed, the glucose resulting from lactose is very efficiently metabolized. Therefore, glucose consumption was estimated, making the difference between glucose added in the medium, and glucose resulting from lactose hydrolysis. Estimation was based on the added sugar mass, given that at the end of the experiments, glucose concentration in the medium was negligible.

Results are shown in Table 7. As shown in this table, both added glucose and glucose resulting from lactose hydrolysis were efficiently consumed during the fermentation runs, although the consumption of glucose from lactoserum, (from lactose) was very low until glucose concentration dropped. In the conditions we used, glucose resulting from lactose hydrolysis represented 12% of total glucose consumption, showing that lactose had contributed to the carbohydrate feedstock. Galactose resulting from lactose hydrolysis was not consumed.

**Table 7: sugar consumption**

| | Standard conditions | No induction | Lactoserum |
|---|---|---|---|
| Total added glucose (g) | 48.6 | 48.5 | 48.5 |
| Total added lactose (g) | - | - | 12.3 |
| Mass of glucose that can be derived by hydrolysis of added lactose (g) | - | - | 6.5 |
| Glucose in medium at end of experiment (g) | n.d | 1 | non detected |
| Galactose in medium at end of experiment (g) | - | - | 5.5 |
| Lactose in medium at end of experiment (g) | - | - | Non detected |
| A: Consumed from added glucose (g) (% of total consumed) | 48,6 (100%) | 47,5 (98%) | 48.5 (88%) |
| B: Consumed from hydrolysed lactose (% of total consumed) | - | - | 6.5 (12%) |
| Total glucose consumed (g) (% of total consumed) | 48.6 | 47.5 | 55.0 (100%) |

## Claims

1. A method for obtaining a casein composition, comprising:
i. providing microorganisms that have been transformed with at least one nucleic acid coding for a casein,
ii. culturing said microorganisms, using a medium comprising lactoserum so as to express and produce casein,
iii. thereby providing a microorganism composition wherein the pH of the composition is equal or above 6.5 and preferably below 9
iv. heating the microorganism composition so as to reduce the amount of the other proteins in soluble fraction of the composition, wherein heating is performed at a temperature at a temperature equal or above 75°C,
v. recovering the soluble fraction from the heated cell composition of iv), thereby obtaining a casein composition in the soluble fraction.

2. The method of claim 1, wherein lactoserum is present in the culture medium at the beginning of the cultivation.

3. The method of claim 1 or 2, wherein lactoserum is added in the medium during exponential phase or stationary phase.

4. The method of any one of claims 1 to 3, wherein another source of carbon is present in addition to lactoserum in the culture medium in ii, wherein this other source of medium is preferably a sugar, notably glucose, fructose, saccharose, possibly present in molasse.

5. The method of any one of claims 1 to 4, wherein the microorganisms are bacteria.

6. The method of claim 5, wherein the bacteria are *E. coli,* in particular *gal+ E. coli.*

7. The method of any one of claims 1 to 6, wherein expression of the at least one nucleic acid is induced by the presence of lactose.

8. The method of claim 7, wherein the at least one nucleic acid is under the control of a promoter fused with the *lac*o repressor, and wherein the microorganisms express the *lac*I repressor.

9. The method of any one of claims 1 to 8, wherein culture of the microorganisms is initiated in presence of a sugar other than lactose, and wherein consumption of lactose present in the lactoserum is initiated at during exponential growth phase, in particular around or after the middle of the exponential phase or at the beginning of the stationary growth phase of the microorganisms.

10. The method of any one of claims 1 to 9, wherein lactoserum is added at a to reach a lactose concentration of at least 1% (w/v).

11. The method of any one of claims 1 to 10, wherein the lactoserum is a lactoserum permeate.

12. The method of any one of claims 1 to 11, wherein lactoserum is acid lactoserup, and wherein pH of culture medium is adjusted around pH7, during microorganism culture.

13. The method of any one of claims 1 to 12, wherein lactose represents at least 2% (w/w, wet weight) of the lactoserum.

14. The method of any one of claims 1 to 13, further comprising washing the microorganism composition to remove the culture medium before heating in step iv) of said method for obtaining a casein composition.

15. The method of any one of claims 1 to 14, further comprising lysing the microorganism composition before iv).

16. The method of any one claims 1 to 15, wherein the microorganisms in the microorganism composition are not lysed and heating in step iv) of said method for obtaining a casein composition. lyses the microorganisms.

17. The method of any one of claims 1 to 16, comprising further processing the soluble fraction by adding at least one step chosen among addition of activated charcoal, membrane filtration, chromatography or casein precipitation, thereby obtaining a casein composition that is purified.

18. The method of any one of claims 1 to 17, wherein heating in in step iv) of said method for obtaining a casein composition is performed at a temperature equal or higher than 95°C.

19. The method of any one of claims 1 to 18, wherein the microorganism composition is flown out of a first container through a device comprising a pipe and wherein the liquid composition is heated in step iv) of said method for obtaining a casein composition. during at least part of the flowing.

20. The method of claim 19, wherein heating in in step iv) of said method for obtaining a casein composition is performed for at most 2 minutes.

21. The method of any one of claims 19 to 20, wherein heating in iv) is performed for at least 2 seconds.

22. The method of any one of claims 19 to 21, wherein heating in in step iv) of said method for obtaining a casein composition is performed for at most 60 seconds.

23. The method of any one of claims 19 to 22, wherein the composition is circulated within a pipe at a flow rate with a comprised between 15 Uh and 30L/h.

24. The method of any one of claims 19 to 23, wherein the composition is delivered to a second container after flowing, optionally after having been recovered by centrifugation and/or filtration after flowing and heating.

25. The method of any one of claims 1 to 24, wherein the casein is selected from beta casein, alpha-S1 casein, alpha-S2 casein and mixture of these caseins, in particular wherein the casein is selected from beta casein, alpha-S1 casein and mixture of these caseins.

26. The method of any one of claims 1 to 25, wherein the microorganisms are transformed with a nucleic acid coding for alpha-casein and another nucleic acid coding for beta casein, wherein expression of the two nucleic acids is induced by lactose.

27. The method of any one of claims 1 to 26, wherein the microorganisms are not transformed with a nucleic acid coding for kappa-casein.

28. The method of any one of claims 1 to 27, wherein the caseins represent at least 60 %, preferably at least 62 %, preferably at least 65 %, preferably at least 67 %, preferably at least 70 % of the total proteins of the soluble fraction.

29. The method of any one of claims 1 to 28, wherein the ratio of casein as compared to the total proteins is increased in the soluble fraction as compared to the ratio in the microorganism composition.

30. A method for obtaining a dairy substitute, comprising performing the method according to any one of claims 1 to 29, mixing the casein composition with at least one other ingredient comprising at least one component selected from the group consisting of proteins, water, calcium, lipids, and carbohydrate to obtain a liquid pre-curd composition (LpCC), and further processing the liquid pre-curd composition (LpCC) to obtain a dairy substitute.

31. The method of claim 30, wherein processing of the LpCC comprises
vi. adding at least one curdling agent to the liquid composition so as to obtain a curd, and
vii. further processing said curd in order to obtain a dairy substitute, wherein the dairy substitute is a cheese substitute or a yoghourt substitute.

32. The method of claim 31, wherein the further step in vi) comprises acidic precipitation of casein.

33. The method of claim 32, wherein the further step of acidic precipitation of casein is conducted by heating at 90°C at pH=4.6.

34. A dairy substitute susceptible to be obtained by a method according to any one of claims 30 to 33.
